# EUROPEAN PATENT SPECIFICATION

(11) **EP 0 873 137 B1**
(45) Date of publication and mention of the grant of the patent: **23.07.2003**
(21) Application number: 97900169.0
(22) Date of filing: 13.01.1997
(51) Int. Cl.: A61K 45/06, A61P 35/00, A61P 29/00

(54) **COMPOSITIONS INCLUDING CALCIUM INFLUX BLOCKERS FOR INHIBITING CELL GROWTH**
ZELLWACHSTUMSHEMMENDE ZUSAMMENSETZUNGEN DIE KALZIUM-ZUFLUSS-BLOCKER ENTHALTEN
COMPOSITIONS CONTENANT DES ANTAGONISTES CALCIQUES POUR INHIBER LA CROISSANCE CELLULAIRE

(30) Priority: 11.01.1996 US 584000
(43) Date of publication of application: 28.10.1998
(73) Proprietor: The Wellesley Hospital Foundation, Toronto, Ontario M4Y 1J3 (CA)
(72) Inventor: BERGER, Stuart, Alan, Toronto, Ontario M6C 1S4 (CA); GOMMERMAN, Jennifer, Lynn, Toronto, Ontario M4J 3A8 (CA)
(74) Representative: VOSSIUS & PARTNER
(86) International application number: CA9700019
(87) International publication number: WO97025063

(56) References cited:
- No relevant documents disclosed

## Description

### FIELD OF THE INVENTION

This invention features the use of a Ca²⁺ influx blocker to induce death of a stimulated cell, and makes available a method for treating a proliferative disease.

### BACKGROUND OF THE INVENTION

Cancer therapies aim, in large part, to eliminate or control the growth of cancer cells while sparing normal tissue. Therefore, much attention has been placed on the identification of characteristics that distinguish cancer cells from normal cells with the goal of targeting these differences with specific therapeutic agents.

The orderly growth and development of cells is controlled, in large part, by the interaction between soluble, secreted ligands and specific receptors on the surface of the stimulated cell (Cell, 61:203-12 [1990]). Not surprisingly, given their important role in controlling cell growth, a number of growth factors or their receptors are known proto-oncogenes including neu, EGF^{R} (v-erb-b), c-fms (CSF-1^{R}), and c-kit, underlying the close relationship in these instances between normal and abnormal growth stimulation. Since growth factors or their receptors are often involved in the genesis and/or maintenance of tumor cells, these molecules may be considered as valid targets for specific therapies aimed at counteracting or neutralizing their stimulatory activity.

The c-kit receptor is an example where Inappropriate expression or mutation has been associated with a number of tumors. In addition to its common expression in myeloid leukemias (Siitonen *et al*., 1994), c-kit is also found in a high proportion of small cell lung cancers (Hida *et al*., 1994), tumors of the female genital tract (Inoue *et al*., 1994), and breast cancers (Hines *et al*., 1995). In many of these cases, the tumor cells express both c-kit and SLF, resulting in an autocrine stimulation that contributes to their tumorigenicity. As well, a common, dominant activating mutation in c-kit has been identified in mastocytomas from human, mouse and rat origin (Tsujimura *et al.*, 1994; Tsujimura *et al*.; 1995; Kanakura *et al*., 1994; Kitayama *et al*., 1995).

Dominant-transforming oncogenes and loss of tumor suppressor function are strongly implicated in many forms of cancer. For instance, several studies have documented mutation, inappropriate expression, overexpression, and autocrine stimulation of growth factor receptors in primary breast cancer cells (Lupu *et al*., 1992; Ethier, 1995; LeJeune *et al*., 1993). Consequently, many investigators are pursuing innovative approaches that specifically target these molecules (Kopreski *et al*., 1996; Fry *et al*., 1995; Levitzki *et al*., 1995). It is also clear that loss of tumor suppressor function can contribute to cancer cell formation through the generation of cells that are no longer subject to physiological mechanisms of cell death. Loss of tumor suppressor function has also been widely documented in breast cancer (Horak *et al*., 1991; Thor *et al*., 1992; Wang *et al*., 1993; Bargou *et al*., 1995; Krajewski *et al*., 1995; Lee, 1995). An important consequence of loss of tumor suppressor function can be resistance to conventional forms of anti-cancer treatment (Lu *et al*., 1996; Korsmeyer, 1995).

Numerous antiproliferative agents are currently in use in the treatment of cancers. For the most part, they are designed to act specifically on rapidly dividing cells. However, since cell division is a normal occurrence in many cell types of the body, toxicity towards normal tissue is often observed, limiting the usefulness and effectiveness of these agents.

In a similar fashion, abnormal or excessive cell activation and proliferation is a characteristic of many inflammatory states. For example, autoimmune diseases represent a situation where cells of the immune system recognize and respond to autoantigens present in the afflicted individual. Current treatments tend to attenuate immune cell responses in a non-specific fashion, resulting in possible susceptibility to infection and other undesirable consequences.

Allergic diseases represent another situation where inappropriate activation signals result in significant morbidity.

In these and other situations, there is therefore a need for therapies with greater specificity towards cells responding to a particular growth or activation signal.

The mobilization of Ca²⁺ from intracellular stores, followed by the influx of extracellular Ca²⁺ through Store-Operated Channels (also called I_{crac} channel) (Hoth *et al.*, 1992; Hoth *et al.*, 1993; Penner *et al*., 1993) is a common signaling event initiated by many growth factor, antigen, Fc, and G-protein-linked receptors. This signal is the consequence of activation of various isoforms of phospholipase C (PLC), leading to the generation of second messengers IP₃ and diacylglycerol (Rhee, 1991). This rapid rise in intracellular Ca²⁺, followed by a gradual decline has been implicated in a number of cellular processes including mitogenesis, activation, and cell movement (Lewis *et al*., 1995).

Although Ca²⁺ influx has been linked to the control of apoptosis, or programmed cell death in a number of cell types, the precise role of Ca²⁺ influx in apoptosis is not understood (Orrenius *et al*., 1992; Nicotera *et al*., 1994; Dowd, 1995). Excessive intracellular Ca²⁺ levels such as those induced by Ca²⁺ ionophore, have been shown to induce apoptosis in a number of experimental systems (Kizaki *et al*., 1989; Tadakuma *et al*., 1990). Apoptosis in splenocytes appears to involve a Ca²⁺-dependent endonuclease (Ribeiro *et al*., 1993) and intracellular Ca²⁺ increases have been linked to apoptosis of both activated T cell hybridomas (Mercep *et al*., 1989) and immature thymocytes (McConkey *et al*., 1989). Recently, Takata *et al*. (Takata *et al*., 1995) showed that surface-IgM-mediated apoptosis of B cells is decreased in the absence of PLC-γ, linking this form of apoptosis with Ca²⁺ mobilization. In contrast to these observations, some cells seem to be protected from apoptosis by Ca²⁺ influx. For instance, IL-3 dependent mast cells and cell lines are protected from growth factor withdrawal mediated apoptosis by addition of Ca²⁺ ionophore (Rodriguez-Tarduchy *et al*., 1992), and programmed neuronal death is also suppressed by increases in intracellular Ca²⁺(Lampe *et al*., 1995).

Certain proteins which mediate both positive and negative effects on apoptosis have been identified. Bcl-2 is a member of a family of related proteins for which the C. elegans ced-9 is the prototype (Reed, 1994). While one class of family members (including Bcl-2) protects cells from apoptotic signals, another class (including bax, a pro-apoptotic member of this family,) promotes apoptosis. It has been suggested that the balance between these two classes determines whether a cell will die apoptotically or survive (Oltvai *et al*., 1993).

Overexpression of the Bcl-2 protein leads to protection from apoptosis induced by a wide variety of agents including radiation, chemotherapeutic drugs, oxidizing agents, and steroids (Korsmeyer, 1995). However other apoptotic signals appear to be Bcl-2 insensitive. These include cell death induced by TNF, Fas activation, activation-induced cell death, anti-IgM treatment of WEHI-231 cells, and superantigen-mediated clonal deletion (Ashwell *et al*., 1987; Smith *et al*., 1989; Jones *et al*., 1990; Sentman *et al.*, 1991; Brown *et al.,* 1992; Cuende *et al*., 1993; Memon *et al*., 1995; Miura *et al*., 1995). In some cases, it has been demonstrated that these apoptotic signals may be neutralized by inhibitors of the interleukin-1β converting enzyme (ICE) family of proteases suggesting involvement of these enzymes in apoptosis (Enari *et al*., 1995; Chinnaiyan *et al*., 1996).

Ketotifen, a known Ca²⁺ influx blocker has been reported to inhibit mast cell activation and proliferation of mononuclear cells at high concentrations (Gushchin *et al*., 1985). It has also been shown that ketotifen can be used to control mast cell-associated symptoms in neurofibroma (Riccardi *et al*. 1987; Riccardi *et al*., 1993). Ketotifen has also been shown to be effective in suppressing the symptoms of systemic mastocytosis (Povoa *et al*., 1991). Ketotifen has been shown to inhibit T cell responses to antigen but not to PHA or tetanus toxoid (Kondo *et al*., 1994). It has been reported that ketotifen can inhibit mitogen-stimulated lymphocyte proliferation (Petrasch *et al*., 1993). High ketotifen concentrations also inhibited T-lymphocyte mitogen- and adenosine triphosphate stimulated increases in intracellular Ca²⁺ in lymphocytes and the U937 human monocyte precursor cell line, respectively. However, in *in vivo* experiments, treatment of healthy volunteers with 1 mg ketotifen b.i.d. for 7 days did not alter the number or subset composition of circulating lymphocytes.

Econazole, another Ca²⁺ influx blocker has been shown to reduce cell viability and cell numbers of NS1/Ag4 myeloma cells at 1µg/ml (Denyer *et al*., 1985).

The compound CAI, which has Ca²⁺ influx inhibitory properties, has been reported to suppress the growth of tumor cells, and the growth of HUVECs in response to FGF (Kohn et al., 1992; Kohn *et al*., 1994a; Kohn *et al*., 1994b).

Inhibition of tumor growth and metastasis with calcium channel blocker compounds is described in U.S. Patent No. 4,690,935 (Taylor *et al*., 1987). Administration of a calcium channel blocker compound and a platinum coordination compound to inhibit tumor growth and metastasis is described in U.S. Patent No. 4,906,646 (Honn *et al*., 1990).

Clotrimazole has been shown to release Ca²⁺ from intracellular stores of 3T3 cells at levels in which it inhibits cell proliferation *in vitro.* The effect of clotrimazole on Ca²⁺ pools was found to be reversible. Clotrimazole was also found to have an inhibitory effect on the number of experimental lung metastases produced in SCID mice by human melanoma cells (Benzaquen *et al*., 1995).

### SUMMARY OF THE INVENTION

A general feature of the present invention is the induction of the death of stimulated cells by a Ca²⁺ influx blocker. The invention, in its various aspects, includes compositions for use in inducing cell death in a mammal, e.g., a human for therapeutic purposes; compositions, e.g., a composition having a Ca²⁺ influx blocker and an agent which normally stimulates cells in which cell death is to be induced; kits of compositions, e.g., a kit having a Ca²⁺ influx blocker and an agent which normally stimulates cells in which cell death is to be induced; use of such compositions or kits of compositions for the manufacture of a medicament for use as anti-inflammatory agents and/or anti-proliferative agents; and use of a Ca²⁺ influx blocker or use of a Ca²⁺ influx blocker and agent in the preparation of a medicament for use in inducing cell death. The invention includes a method for diagnosing the susceptibility of diseased animals to treatment with a Ca²⁺ influx blocker or the combination of a Ca²⁺ influx blocker and agent which normally stimulates cells in order that the treatment can induce cell death. The invention includes a method for screening potential compounds as Ca²⁺ influx blockers.

In the context of this invention, an agent or factor or ligand or other agonist which normally stimulates a cell or cell receptor is one which stimulates the cell or receptor. Such stimulation generally promotes growth, survival and/or activation of the cell. In other words, according to the present invention, cell death is actually induced in a stimulated cell. According to a particular example, the details of which are described below, mast cells which are normally stimulated when cultured in the presence of SLF are induced to die when cultured in the presence of a combination of SLF and a Ca²⁺ influx blocker. In general such normal stimulation is accompanied by Ca²⁺ influx into the cell.

Preferred Ca²⁺ influx blockers are non-voltage-gated influx blockers.

Preferred subjects of treatment are human.

Death of cells is induced in a mammal in need thereof, wherein the cells are capable of being stimulated by a factor with accompanying Ca²⁺ influx by an effective amount of a Ca²⁺ influx blocker and an effective amount of such a factor.

In a mammal having cells that are subject to stimulation by a ligand of a receptor of a said cell by an agent, with accompanying Ca²⁺ influx, death of the cells is induced by an effective amount of a Ca²⁺ influx blocker and an effective amount of such a ligand.

Death of cells of a mammal in need thereof is induced, wherein such a cell has a receptor which, upon binding by an agent results in calcium influx into the cell and activation of phospholipase C, by an effective amount of a Ca²⁺ influx blocker and an effective amount of such an agent.

Death of cells of a mammal in need thereof is induced, wherein the cells are capable of being stimulated by a factor with accompanying Ca²⁺ influx, by an effective amount of a non-voltage gated Ca²⁺ influx blocker and an effective amount of such a factor.

The factor or agent, etc., can be for example an agonistic antibody to a receptor of the cells, or a natural ligand of a receptor of the cells.

The cells can be mast cells, monocytes, macrophages, fibroblasts, T cells, B cells, basophils or cancer cells.

The mammal can be in need of treatment of a proliferative disease, for example, it might be a human suffering from cancer, particularly breast cancer, or the patient might be suffering from an allergy or from an autoimmune disorder.

A cell (or group of cells) in which death is to be induced can have, for example, a surface receptor selected from the group c-kit receptor or a mutant c-kit receptor, a T cell receptor, CD3, FcγRII, FcγRIII, FcεRI, a G-protein-linked receptor, a receptor for bombesin, a receptor for a gastrin releasing peptide, a receptor for bradykinin, a receptor for carbachol, a receptor for muscarinic molecule, a receptor for CCK-8, a receptor for vasopressin, a receptor for neurokinin, a receptor for substance P, a receptor for a purinergic molecule, a receptor for TGF-α, a receptor for EGF, a receptor for heregulin, a receptor for erbB1, a receptor for erbB2, a receptor for erbB3, a receptor for erbB4, a receptor for PDGF, a receptor for SLF, a receptor for FLT-3 ligand, a receptor for a basic FGF, a receptor for an acidic FGF receptor for enothelin, a receptor for NGF, a receptor for VEGF, and a receptor for HGF.

The agent or factor to be administered to said cells can be selected from the group SLF, TGF-α, EGF, heregulin, agonists to erbB1, agonists to erbB2, agonists to erbB3, agonists to erbB4, PDGF A, PDGF B, FLT-3 ligand, basic FGF, acidic FGF, enothelin, NGF, VEGF, HGF, agonists to a TCR, agonists to CD3 receptor, agonists to FcγRII receptor, agonists to FcγRIII receptor, agonists to FcεRI receptor, agonists of G-protein-linked receptors, bombesin, gastrin, gastrin releasing peptide, bradykinin, carbachol, muscarinic receptor agonists, CCK-8, vasopressin, neurokinins, substance P, purinergic receptor agonists, ATP, adenosine, and 1,25-dihydroxyvitamin D₃, and combinations thereof.

The cell can be a hyperactive immune cell and the method can include an antibody and the factor can be an antigen to the antibody, for example. The cell can be a hyperactive immune cell in which an IgE is bound to the cell and the factor can be an agonist to the IgE, for example.

The cell can include an IgE receptor and the factor can be an antibody to the receptor.

The cells can be basophils or mast cells.

The Ca²⁺ influx blocker and the factor, ligand, etc. can be administered separately.

According to a preferred embodiment, the Ca²⁺ influx blocker is to be administered prior to the agent.

According to another preferred embodiment, the agent/factor, etc. and the Ca²⁺ influx blocker are to be administered in a single step.

Preferably, stimulation of the cells by such an agent in the absence of a Ca²⁺ influx blocker normally promotes growth, survival and/or activation of the cells.

In a particular embodiment, the cells express a c-kit receptor wherein an effective amount of a c-kit ligand is to be administered to the mammal.

The patient can be suffering from leukemia, tumor growth or lung cancer.

A factor of the invention can be a growth factor which binds to a receptor of a cell, which binding normally causes proliferation of the cell.

The patient can be suffering from cancer or hyperplasia.

The factor can be a protein.

The factor can be a survival factor which binds a receptor of a said cell, which binding, in the absence of a said Ca²⁺ influx blocker normally enhances the longevity of the cell.

According to a particular embodiment, the cells are haematopoietic progenitor cells.

The factor can be an activation factor which binds to a receptor of a said cell, which binding, in the absence of a said Ca²⁺ influx blocker normally causes activation of the cell.

The Ca²⁺ influx blocker can be any one or more of Ni²⁺, ketotifen, econazole, tenidap, CAI, Cd²⁺, Co²⁺, La³⁺, Mn²⁺, SKF-96365 and cromolyn, or a combination thereof.

Death of constitutively activated cells of a mammal in need thereof, is induced by an effective amount of a Ca²⁺ influx blocker to the mammal. Preferably, the activation results in a concentration of Ca²⁺ elevated above levels in said cells when in an unactivated state. The constitutive activation can be the result of the presence of a mutant c-kit receptor. The patient can be suffering from mastocytosis.

Death of cells of a mammal in need thereof in which the cells are subject to autocrine stimulation, is induced by an effective amount of a Ca²⁺ influx blocker to the mammal. The Ca²⁺ influx blocker can be Ni²⁺.

According to another embodiment, the invention is a pharmaceutical composition for use as an anti-proliferative agent to inhibit cell growth, comprising a Ca²⁺ influx blocker and a factor which normally binds to a receptor of said cell so as to cause Ca²⁺ influx into the cell.

The composition can be one in which binding of such a factor to the receptor normally results in activation of phospholipase C. The composition can include a Ca²⁺ influx blocker which is a non-voltage-gated Ca²⁺ influx blacker. The factor can be an agonistic antibody to the receptor. The factor can be a natural ligand of the receptor. The factor might be selected from the group SLF, TGF-α, EGF, heregulin, agonists to erbB1, agonists to erbB2, agonists to erbB3, agonists to erbB4, PDGF A, PDGF B, FLT-3 ligand, basic FGF, acidic FGF, enothelin, NGF, VEGF, HGF, agonists to a TCR, agonists to CD3 receptor, agonists to FcγRII receptor, agonists to FcγRIII receptor, agonists to FcεRI receptor, agonists of G-protein-linked receptors, bombesin, gastrin, gastrin releasing peptide, bradykinin, carbachol, muscarinic receptor agonists, CCK-8, vasopressin, neurokinins, substance P, purinergic receptor agonists, ATP, adenosine, and 1,25-dihydroxyvitamin D₃, and combinations thereof. In a particular embodiment the composition includes SLF as a factor.

A composition might include an antigen of a surface antibody of an immune cell.

The composition can include a factor which is an agonist to an IgE.

The composition can include a factor, binding of which said factor to the receptor normally, in the absence of a said Ca²⁺ influx blocker, promotes growth, survival and/or activation of a said cell.

According to another aspect, the invention is a kit of pharmaceutical compositions for use in inhibition cell growth in a mammal, the kit comprising a Ca²⁺ influx blocker and a factor which normally binds to a receptor of said cell so as to cause Ca²⁺ influx into the cell. The factor can be one in which binding thereof to the receptor normally results in activation of phospholipase C.

The Ca²⁺ influx blocker can be a non-voltage-gated Ca²⁺ influx blocker.

A factor of the composition can be an agonistic antibody to the receptor. The factor can be a natural ligand of the receptor. The factor can be selected from the group SLF, TGF-α, EGF, heregulin, agonists to erbB1, agonists to erbB2, agonists to erbB3, agonists to erbB4, PDGF A, PDGF B, FLT-3 ligand, basic FGF, acidic FGF, enothelin, NGF, VEGF, HGF, agonists to a TCR, agonists to CD3 receptor, agonists to FcγRII receptor, agonists to FcγRIII receptor, agonists to FceRI receptor, agonists of G-protein-linked receptors, bombesin, gastrin, gastrin releasing peptide, bradykinin, carbachol, muscarinic receptor agonists, CCK-8, vasopressin, neurokinins, substance P, purinergic receptor agonists, ATP, adenosine, and 1,25-dihydroxyvitamin D₃, and combinations thereof. Particularly, the factor can be SLF.

The factor can be an antigen of surface antibody of an immune cell.

The factor can be an agonist to an IgE.

The factor can be one for which wherein thereof to the receptor normally, in the absence of a said Ca²⁺ influx blocker, promotes growth, survival and/or activation of a said cell.

A kit of pharmaceutical compositions of the present invention can, correspondingly, be used as an anti-proliferative agent or as an anti-inflammatory agent.

The invention includes the use of a Ca²⁺ influx blocker and a factor which normally binds to a receptor of a cell so as to cause Ca²⁺ influx into the cell in the preparation of a medicament for use as an agent to inhibit growth of said cells.

The invention includes the use of a Ca²⁺ influx blocker and a factor of the invention in the preparation of a medicament for use as an agent to inhibit growth of said cells wherein binding of a said factor to the receptor normally results in activation of phospholipase C.

The invention includes the use of a Ca²⁺ influx blocker and a factor of the present invention in the preparation of a medicament for use as an agent to inhibit growth of said cells wherein said Ca²⁺ influx blocker is a non-voltage-gated Ca²⁺ influx blocker.

According to a particular embodiment, the invention includes the use of a Ca²⁺ influx blocker and a factor of the invention in the preparation of a medicament for use as an agent to inhibit growth of said cells wherein said factor is an agonistic antibody to the receptor.

A particular embodiment includes the use of a Ca²⁺ influx blocker and a factor of the invention in the preparation of a medicament for use as an agent to inhibit growth of said cells wherein said factor is a natural ligand of the receptor.

Particular embodiments include the use of a Ca²⁺ influx blocker and a factor of the present invention in the preparation of a medicament for use as an agent to inhibit growth of said cells wherein said factor is selected from the group SLF, TGF-α, EGF, heregulin, agonists to erbB1, agonists to erbB2, agonists to erbB3, agonists to erbB4, PDGF A, PDGF B, FLT-3 ligand, basic FGF, acidic FGF, enothelin, NGF, VEGF, HGF, agonists to a TCR, agonists to CD3 receptor, agonists to FcγRII receptor, agonists to FcγRIII receptor, agonists to FcεRI receptor, agonists of G-protein-linked receptors, bombesin, gastrin, gastrin releasing peptide, bradykinin, carbachol, muscarinic receptor agonists, CCK-8, vasopressin, neurokinins, substance P, purinergic receptor agonists, ATP, adenosine, and 1,25-dihydroxyvitamin D₃, and combinations thereof. The factor can be, in particular, SLF.

A preferred embodiment includes the use of a Ca²⁺ influx blocker and a factor of the invention in the preparation of a medicament for use as an agent to inhibit growth of said cells wherein said factor is an antigen of surface antibody of an immune cell.

A preferred embodiment includes the use of a Ca²⁺ influx blocker and a factor of the invention in the preparation of a medicament for use as an agent to inhibit growth of said cells wherein said factor is an agonist to an IgE.

Another embodiment includes the use of a Ca²⁺ influx blocker and a factor of the invention in the preparation of a medicament for use as an anti-proliferative agent wherein binding of a said factor to the receptor normally, in the absence of a said Ca²⁺ influx blocker, promotes growth, survival and/or activation of a said cell.

According to another embodiment, the invention is a method for diagnosing susceptibility of diseased mammalian cells to treatment with a Ca²⁺ influx blocker, or a blocker and factor which further activates receptors of the cells, comprising:
assaying whether a sample of tissue contains a level of PLC activity which is elevated in comparison to normal tissue; wherein,
an elevated level of activated PLC indicates the cells are likely to be susceptible to said treatment.

Such a method can also include using a sample of diseased tissue.

Further the method can include an assaying step in which includes monitoring the amount of a PLC substrate which reacts in the presence of PLC obtained from a predetermined amount of said tissue. The method can also include isolating the PLC from the predetermined amount of said tissue by an enzyme-linked immunosorbent assay. The PLC substrate can be [³H]-PIP₂.

In another embodiment, the invention is a method for screening an agent as a Ca²⁺ influx blocker, the method comprising the steps of:
culturing a first group of cells in the presence of the agent, wherein the cells have an activated receptor which promotes influx of Ca²⁺ into the cells;
culturing a second group of cells in the presence of the agent, wherein the cells of the second group lack an activated receptor which promotes influx of Ca²⁺ into cells of the second group;
ascertaining whether growth of the first group of cells is less than a first predetermined level corresponding to growth of cells of the first group in the absence of the agent; and
ascertaining whether growth of the second group of cells is substantially the same as a second predetermined level corresponding to growth of cells of the second group in the absence of the agent; wherein,
growth of the first group of cells less than the first predetermined level and growth of the second group of cells substantially the same as the second predetermined level indicates that said agent is a Ca²⁺ blocker.

The receptor of the first group of cells can be constitutively activated, or the receptor of the first group of cells is subject to autocrine stimulation, or the receptor of the first group of cells is activated by an exogenous agent.

The agent can be a natural ligand of the receptor of the first group of cells. The receptor of the first group of cells and the receptor of the second group of cells can be the same receptor.

In particular embodiments, the receptor is selected from the group of c-kit receptor, a receptor for EGF and a receptor for FGF.

Preferably, the cells of the first group and the cells of the second group are human cells.

Preferably, the cells of the first group are mast cells, the cells of the second group are mast cells, the receptor is the c-kit receptor and the first group of cells is cultured in the presence of SLF.

According to a particular embodiment, the first group of cells is transfected with said receptor of the first group of cells.

### BRIEF DESCRIPTION OF THE DRAWINGS

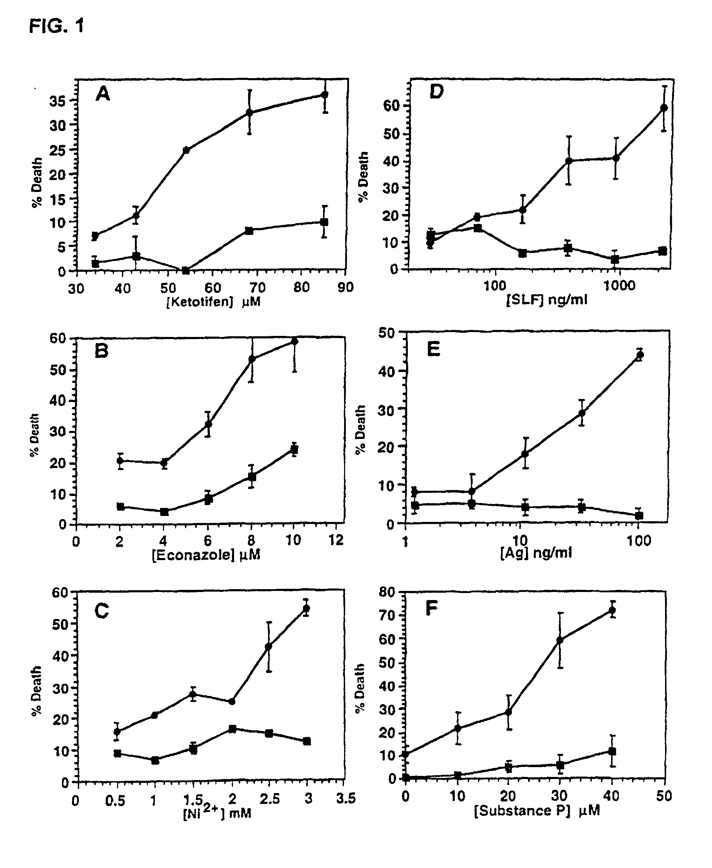

Figures 1A to 1F show induction of death in BMMC through the combination of growth or activation signals with Ca²⁺ influx blockers. Cytotoxicity assays were performed as described in Materials and Methods. In Figures 1A to 1C, BMMCs were incubated with either SLF (500ng/ml in all cases) (circles) or IL-3 (25% WEHI-3 conditioned medium in all cases) (squares) with the indicated amounts of ketotifen (Figure 1A), econazole (Figure 1B) or Ni²⁺ (Figure 1C). In Figure 1D, BMMCs were incubated with IL-3, and the indicated amounts of SLF. Plotted circles also included 2.5mM Ni²⁺ and plotted squares included no Ni²⁺. In Figure 1E, BMMCs were coated with IgE anti-DNP monoclonal SPE-7, then incubated with IL-3 plus the indicated amounts of DNP-HSA (Ag), with 5µM econazole (circles) or without econazole (squares). In Figure 1F, BMMCs were incubated with IL-3 plus the indicated amounts of Substance P, with 5µM econazole (circles) or without econazole (squares). In all cases, the proportion of dead cells was determined after 24 hours in culture by counting cells that could or could not exclude Trypan blue. Error bars represent the standard error determined from triplicate measurements.

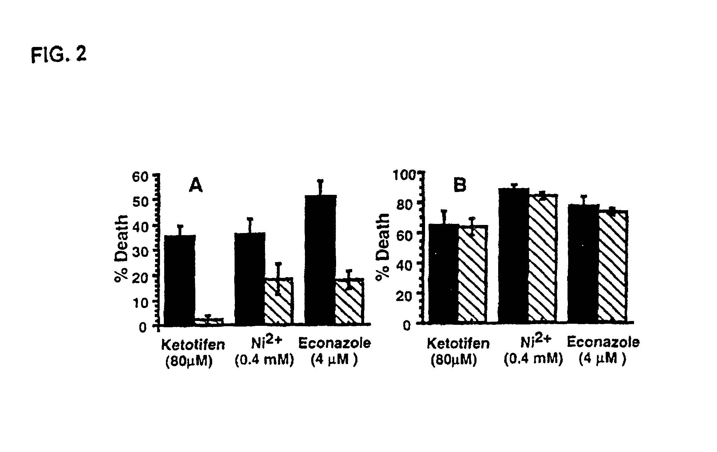

Figures 2A and 2B show induction of cell death in 32D-kit or p815 cells with SLF plus Ca²⁺ influx blockers. 32D-kit (Figure 2A) or p815 (Figure 2B) cells were incubated with either SLF (solid bars) or IL-3 (striped bars) plus the indicated concentration of Ca²⁺ influx blocker. Cell death was determined by Trypan Blue exclusion after 18 hours in culture.

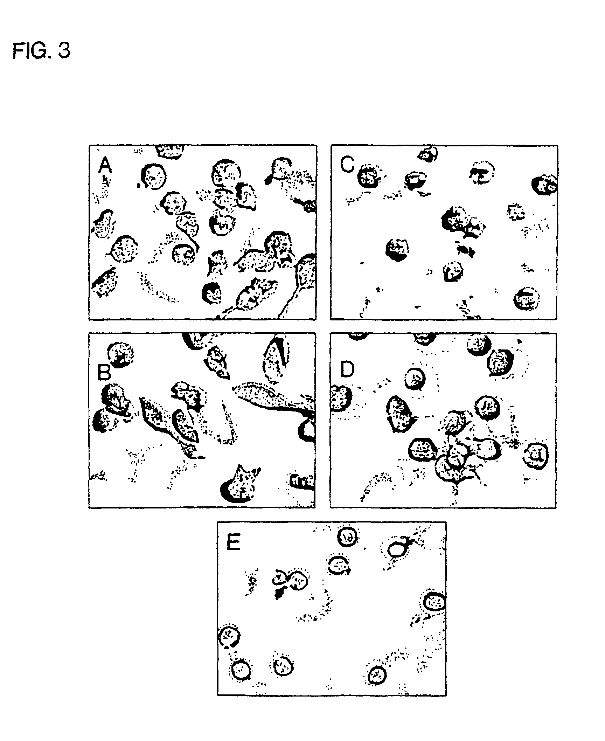

Figures 3A to 3E show morphology of 32D-kit cells incubated with various factors and Ca²⁺ influx blockers, 32D-kit cells were incubated with SLF alone (Figure 3A), IL-3 alone (Figure 3B), 5µM econazole and SLF (Figure 3C), or 5µM econazole and IL-3 for 18 hours (Figure 3D). In Figure 3E, the cells were incubated with no added factor. Cells were photographed under phase contrast at 400X magnification.

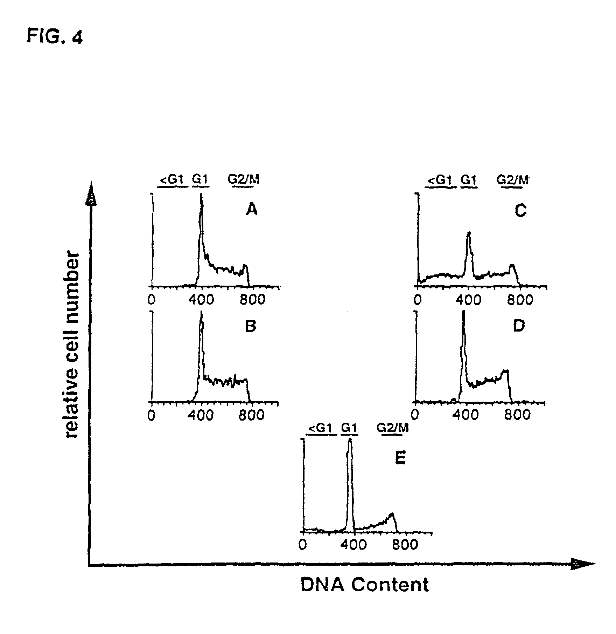

Figure 4 shows plots of relative number of 32D-kit cells (y-axis) as a function of cellular DNA content. 32D-kit cells were incubated with SLF alone (Figure 4A), IL-3 alone (Figure 4B), in 8µM econazole and SLF (Figure 4C), or in 8µM econazole and IL-3 (Figure 4D) for 18 hours. In Figure 4E, cells were incubated with no added factor. Cells were stained with propidium iodide as described in Materials and Methods and then analyzed by flow cytometry for DNA content.

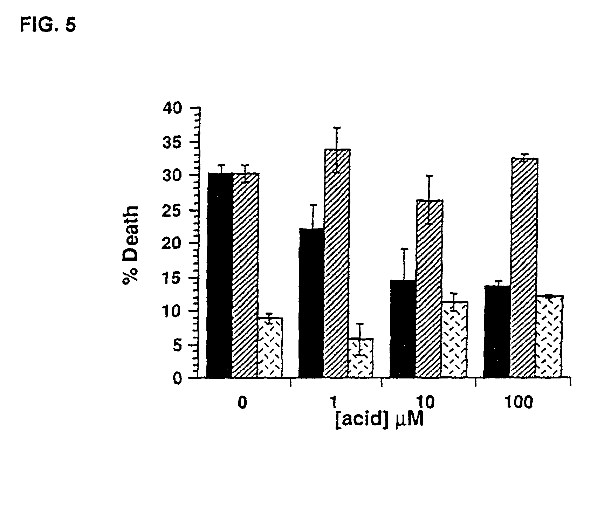

Figure 5 shows oleic acid protection of 32D-kit cells from apoptosis induced by SLF and Ca²⁺ influx blocker. 32D-kit cells were incubated for 18 hours with SLF and 5µM econazole and oleic acid (solid bars), or with SLF and 5µM econazole and elaidic acid (hatched bars), or IL-3 and 5 µM econazole and oleic acid (stippled bars). The proportion of dead cells was determined by Trypan Blue exclusion.

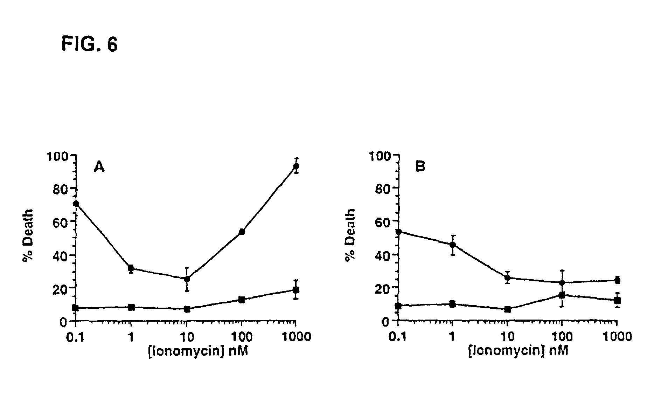

Figures 6A and 6B show the effect of ionomycin on apoptosis induced by SLF and Ca²⁺ influx blacker. In Figure 6A, 32D-kit cells were incubated for 18 hours with 5µM econazole and the indicated amounts of ionomycin in the presence of either SLF (circles) or IL-3 (squares). In Figure 6B, 32D-kit-Bcl-2 cells were incubated for 18 hours with 5µM econazole and the indicated amounts of ionomycin in the presence of either SLF (circles) or IL-3 (squares). The proportion of dead cells was determined by Trypan Blue exclusion.

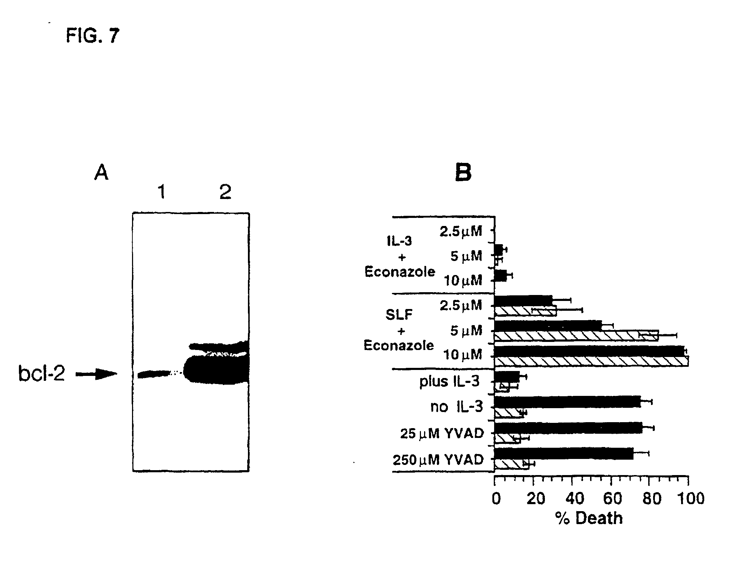

Figure 7A shows Western blot of cell Iysates from 32D-kit (lane 1) or 32D-kit-Bcl-2 (lane 2) cells. Cells were lysed as described in Materials and Methods and separated by SDS-PAGE, transferred to nitrocellulose and probed with anti-Bcl-2 antibodies.

Figure 7B shows the effect of Bcl-2 overexpression on apoptosis induced by SLF plus Ca²⁺ influx blocker, 32D-kit (solid bars) or 32D-kit-Bcl-2 (striped bars) cells were incubated for 18 hours with SLF or IL-3 plus 2.5, 5 or 10µM econazole and the proportion of dead cells was determined by Trypan Blue exclusion. In additional cultures, IL-3 alone or no factor was added and cell viability was similarly tested. The effect on cell viability of adding 25 or 250 µM YVAD-CHO was also evaluated in separate cultures in which no additional factors were added.

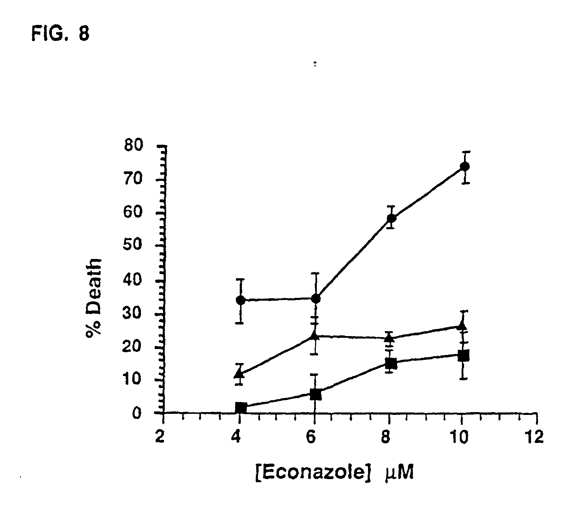

Figure 8 shows the effect of YVAD-CHO on apoptosis induced by SLF and Ca²⁺ influx blocker. 32D-kit cells were incubated with either SLF in the absence of YVAD-CHO (circles), or with SLF in the presence of 25 µM YVAD-CHO. (triangles), or IL-3 (squares) with the indicated amounts of econazole. The proportion of dead cells was evaluated by Trypan Blue exclusion after 18 hours in culture

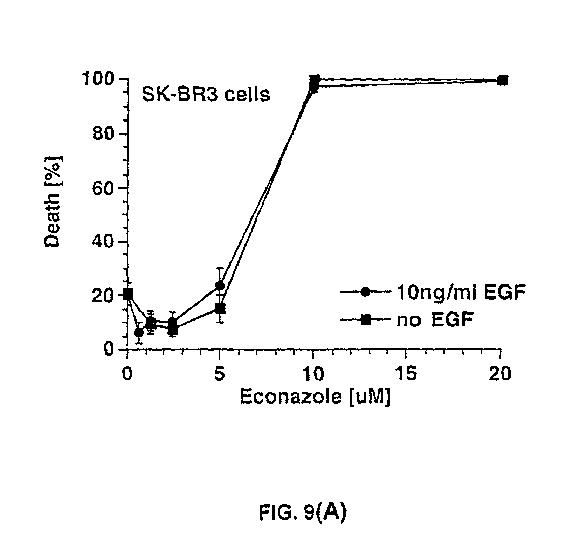

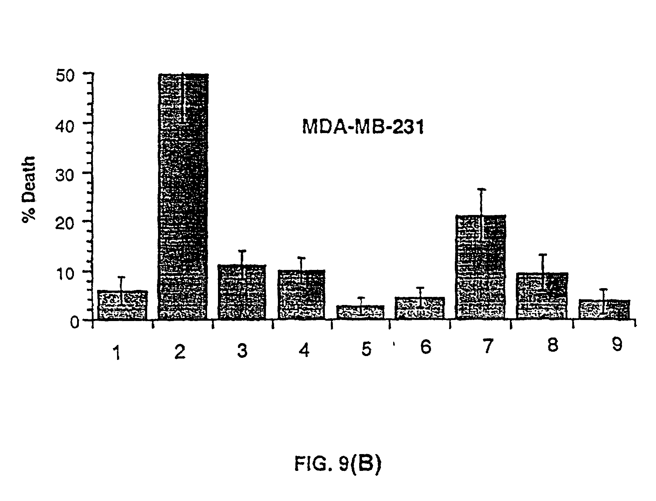

Figure 9A shows apoptosis induced by a Ca²⁺ influx blocker in cancer cells having a continuously activated receptor. 2.5 X 10⁴ SK-BR3 cells (ATCC cat. # HTB-30) were incubated in the wells of a 96-well plate in 0.1ml RPMI plus 0.5% FBS with (circles) or without (squares) 10ng/ml EGF (epidermal growth factor) and with the indicated concentrations of econazole for 18 hours. The proportion of dead cells was evaluated by Trypan Blue exclusion after 18 hours in culture.

Figure 9B shows apoptosis induced by Ca²⁺ blocker in autocrine stimulated MDA-MB-231 breast cancer cells under various conditions. Bar 1: control; Bar 2: 20µM econazole; Bar 3: 20µM econazole and 10µM YVAD-CHO (ICE inhibitor); Bar 4: 20µM econazole and 100 µM YVAD-CHO; Bar 5: 20µM econazole and 0.1 nM ionomycin (calcium ionophore); Bar 6: 20µM econazole and 10 nM ionomycin; Bar 7: 20µM econazole and 1 µM ionomycin; Bar 8: 20µM econazole and 10 µM oleic acid (PLC activation inhibitor); and Bar 9: 20µM econazole and 100 µM oleic acid.

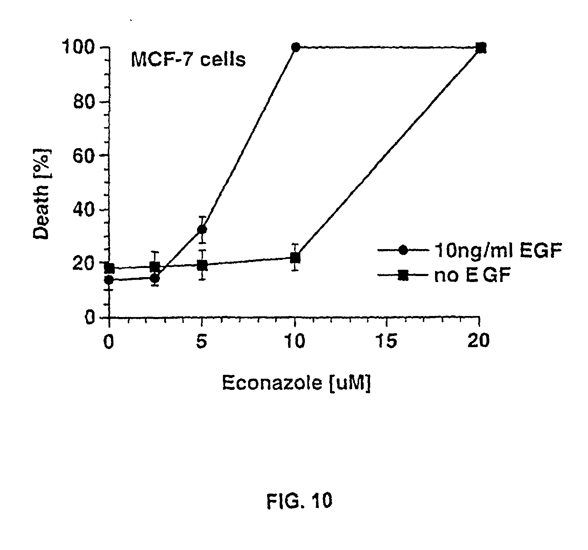

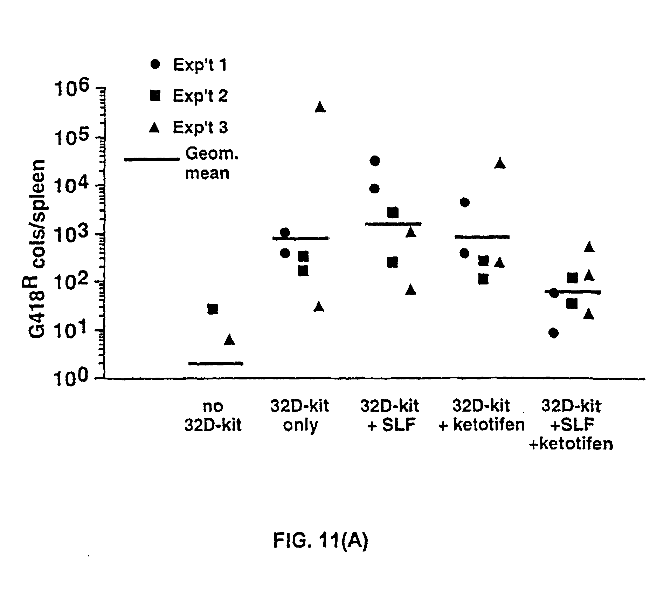

Figure 10 shows apoptosis induced by EGF and Ca²⁺ influx blocker in cells derived from human breast carcinoma. 2.5 X 10⁴ MCF-7 cells were incubated in the wells of a 96-well plate in 0.1ml DMEM plus 0.5% FBS with (circles) or without (squares) 10ng/ml EGF and with the concentrations of econazole indicated for 18 hours. The proportion of dead cells was evaluated by Trypan Blue exclusion after 18 hours in culture.

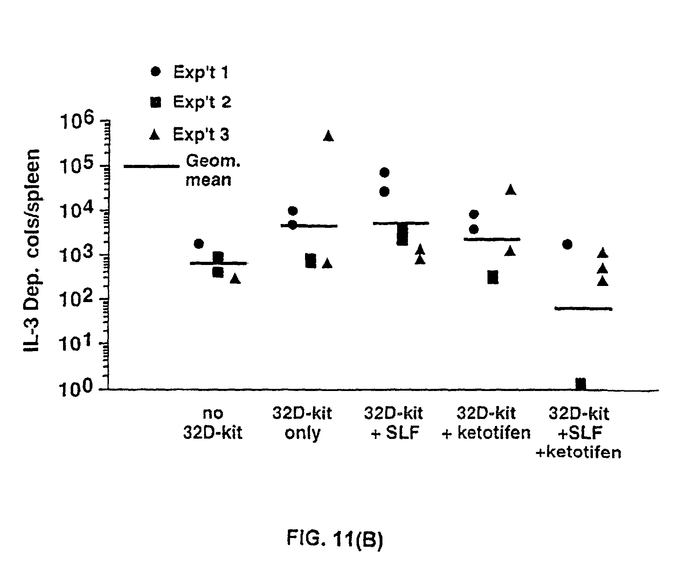

Figure 11A shows the effect of Ca²⁺ influx blocker and activating factor on leukemia cells in mice in which SLF was administered intravenously. Mice were inoculated intravenously with 1 X 10⁷ G418-resistant 32D-kit cells. Between 3 and 5 weeks following the inoculation (Experiments 1 & 3; 5 weeks, experiment 2; 3 weeks), mice were given 100mg/kg ketotifen by oral gavage. Four hours following the ketotifen treatment, mice were injected with 15µg SLF intravenously. Two hours later the mice were given a further 100mg/kg ketotifen p.o. The following day, spleens were removed and assayed for the presence of leukemic cells by plating cells in 0.3% agar in growth medium containing 20% WEHI-3 conditioned medium and 1 mg/ml G418 to limit detection to the G418-resistant leukemic cells. Seven days later, colonies were enumerated.

Figure 11B is similar to Figure 11A, but in this case spleen cells were assayed for the presence of all cells capable of forming colonies in agar in response to IL-3, to include leukemic as well as normal cells.

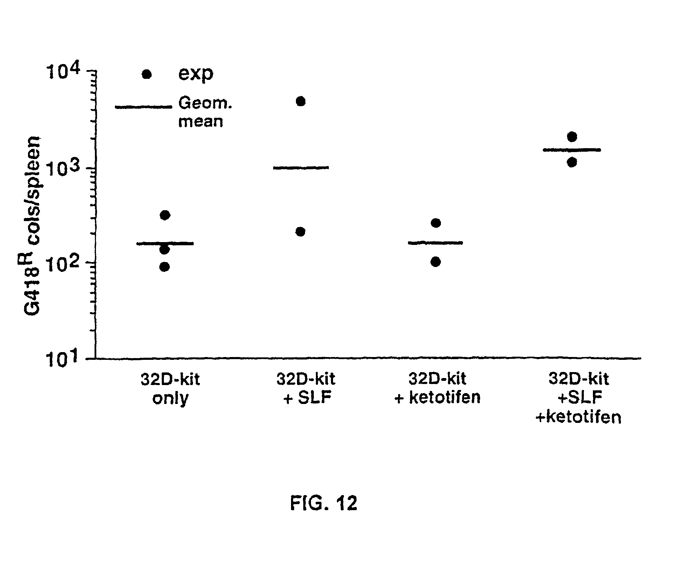

Figure 12 shows the effect of Ca²⁺ influx blocker and activating factor on leukemia cells in mice in which SLF was administered subcutaneously. Mice were inoculated intravenously with 1 X 10⁷ 32D-kit cells. Three weeks following inoculation, the mice were given 100mg/kg ketotifen by oral gavage. Four hours following the ketotifen treatment, the mice were injected with 15µg SLF subcutaneously. Two hours following the injection, mice were given a further 100mg/kg ketotifen p.o. The following day, spleens were removed and assayed for the presence of leukemic cells by plating cells in 0.3% agar in growth medium containing 20% WEHI-3 conditioned medium and 1mg/ml G418 to limit detection to the G418-resistant leukernic cells. Seven days later, colonies were enumerated.

### DESCRIPTION OF PREFERRED EMBODIMENTS

### Materials and Methods

***Cells.*** Bone marrow derived mast cells (BMMC) were generated as described previously (Berger *et al*., 1994). They were cultured in OPTI-MEM (Gibco, Burlington, ON) supplemented with 10% heat-inactivated FBS and 2% WEHI-3 supernatant as a source of IL-3. 32D-kit cells (gift from Dr. Mark Minden, Ontario Cancer Institute) are an IL-3-dependent myelomonocytic cell line expressing c-kit (Hu *et al*., 1995). 32D-kit cells were grown in RPMI (Gibco) supplemented with 10% heat-inactivated FBS, 2% WEHI-3 supematant, and 1 mg/ml G418 (Gibco). The p815 cell line is a murine mastocytoma cell line. P815 cells were grown in RPMI supplemented with 10% heat-inactivated FBS. Bcl-2 gp+e NIH 3T3 packaging cells were grown in Dulbecco's modified Eagle's medium (DMEM - Gibco) and supplemented with 10% FBS and 2 µg/ml puromycin (Sigma, St. Louis, Mo). All cell cultures also contained 55µM β-mercaptoethanol and antibiotics (both Sigma). SK-BR3, MDA-MB231 and MCF-7 cells were obtained from ATCC. SK-BR3 and MDA-MB231 cells were cultured in RPMI medium supplemented with 10% heat inactivated fetal bovine serum. MCF-7 cells were cultured in DMEM medium supplemented with 10% heat inactivated fetal bovine serum and 10 µg/ml human insulin (Sigma).

***Generation of Bcl-2 over-expressing 32D-kit cells***. gp + e bcl-2 retroviral producer cells were a gift from Dr. Y. Ben-David (Toronto) and contain an LXSN-based retroviral vector expressing genes for both puromycin resistance and murine bcl-2. For infection, a confluent layer of gp + e packaging cells were co-cultured with 32D-kit cells for 24 hours. Non-adherent 32D-kit cells were then removed, cultured for 48 hours and then selected for bcl-2 over-expressing cells in the presence of 2µg/ml puromycin.

***Production of recombinant SLF***. Recombinant murine Steel Factor (SLF) was produced in soluble form in E. coli using the pFLAG.ATS, IPTG-inducible secretion expression vector (InterScience, Markham, ON). This vector includes an eight amino acid N-terminal FLAG epitope (InterScience). E. coli containing the pFLAG.ATS.SLF plasmid were incubated overnight at 37°C in Luria Broth (Gibco) with 100 µg/ml ampicillin (Sigma). This culture was then diluted 20-fold and grown to an OD₆₀₀ of 0.4-0.5 before being induced with 33mg/L IPTG (Gibco). The cultures were then incubated overnight at 37°C, and the cells were pelleted at 10,000 rpm for 20 minutes. The bacterial supernatant was passed through a 0.22 micron filter and stored at -80°C with 1mM CaCl₂ and 100µM PMSF. FLAG-SLF was affinity-purified on a column of Anti-FLAG M1 mouse monoclonal antibodies covalently attached to agarose gel (InterScience). This monoclonal binds the FLAG epitope in a Ca²⁺-dependent manner enabling elution by chelating excess Ca²⁺ with EDTA. The column was first equilibrated with 30 ml PBS + 1mM CaCl₂ and bacterial supernatants were passed over the column 3 times. The column was washed extensively with PBS + 1mM CaCl₂ and the FLAG-SLF fusion protein was eluted with 6 aliquots of 1 ml of PBS + 2mM EDTA. These were collected, concentrated, dialysed against PBS and checked for purity by silver stain.

***Other reagents.*** Monoclonal murine dinitrophenyl (DNP) - specific IgE, clone SPE-7 as well as albumin - human DNP-HSA antigen were obtained from SIGMA. Substance P, all Ca²⁺ channel blockers, ionomycin, oleic and elaidic acids EGF, and bFGF were also obtained from Sigma. Ionomycin was stored as a 1mM solution in DMSO at -20°C. Oleic and elaidic acids were stored in de-gassed ethanolic solutions at concentrations of 1M and 100mM respectively, sealed under a source of nitrogen gas and stored at -20°C. YVAD-CHO ICE protease inhibitor peptide was obtained from Amersham (Arlington Heights, IL) and stored as a 1mM solution in DMSO at -20°C.

***Cell death assays.*** 2.5 X 10⁴ BMMCs, 32D-kit or P815 cells were placed in 96-well flat bottom plates in a volume of 0.1 ml RPMI containing 0.5% FBS. Cells were supplemented with either SLF, Substance P or IL3 plus Ca²⁺ channel blocker. In the case of adding IgE, cells were incubated with 10 µg/ml SPE-7 for 45 minutes at 4°C followed by 3 washes with RPMI, 0.5% FBS before plating in 96 well plates and adding 100 ng/ml DNP-HSA. The proportion of dead cells was determined after 18 or 24 hours in culture by counting cells that could or could not exclude Trypan blue.

***Analysis of DNA content*.** 1.25 X 10⁶ cells were incubated for 18 or 24 hours as described above. Cells were spun down and resuspended in Vindelov's reagent: 3.4mM Tris - pH 8, 75 µM Propidium Iodide (from Sigma), 0.1% NP-40, 700 u/l RNAse (Sigma) and 10 mM NaCl. Cells were then analysed by flow cytometry.

***Western Blotting.*** 1 X 10⁶ 32D-kit and 32D-kit-bcl2 cells were washed in PBS and resuspended in lysis buffer containing 1% NP-40, 10% glycerol in TBS with inhibitors: 500 µM sodium-orthovanadate, 10 µg/ml aprotinin, 10 µg/ml leupeptin and 1 mM PMSF (all Sigma) and incubated at 4°C for 20 minutes. Lysates were centrifuged at 12,000 rpm for 10 minutes, and the supernatant was added to an equal volume of sample buffer with β2-mercaptoethanol. Samples were separated by 12% SDS-PAGE and transferred to nitrocellulose. The blot was blocked with 5% skim milk powder, 0.1% TWEEN-20 (ICN, Aurora OH) in PBS and probed with anti-bcl-2 antibodies (U.B.I., Lake Placid, NY). The Western blot was developed with chemiluminescence reagents (Amersham).

***Mice.*** For the *in vivo* 32D-kit experiments, mice were male C3H/ he mice 6 to 8 weeks old and obtained from Charles River Laboratories, Boston, Massachusetts. Mice were inoculated intravenously with 1 x 10⁷ leukemic cells. Between 3 and 5 weeks following the inoculation, mice were given 100 mg/kg ketotifen (Sigma) in a volume of 0.4 ml water by oral gavage, Following the first ketotifen treatment, mice were injected intravenously or subcutaneously with 15 µg SLF in PBS. A second ketotifen treatment followed administration of SLF.

### Experiment 1: Ca²⁺ channel blockers convert activation signals to death signals.

Mast cells are normally stimulated to proliferate by SLF, the ligand for the c-kit receptor tyrosine kinase. However, as shown in Figures 1A-C, this proliferative signal is converted to a death signal if the cells are co-incubated with a Ca²⁺ Influx blocker such as ketotifen, econazole or Ni²⁺. In contrast, death is not observed when mast cells are incubated with a Ca²⁺ influx blocker and IL-3. Simultaneous incubation of mast cells with SLF, Ca²⁺ influx blocker, and IL-3 still results in cell death (Figure 1D), indicating that IL-3 is not protective for this particular death signal. Ca²⁺ influx blockers on their own do not accelerate mast cell death due to withdrawal of growth or survival factors (not shown). Induction of cell death by SLF plus Ca²⁺ influx blocker increases with increasing concentrations of SLF. This indicates that there is a signaling property associated with SLF, but not IL-3, that is required to induce this form of cell death in conjunction with a blocker. The effect of the Ca²⁺ influx blocker is not simply to counteract or neutralize the stimulatory properties of SLF. Rather, a blocker combines with a critical signal generated by SLF to convert an activation pathway to a cell death pathway.

Although both SLF and IL-3 are mitogenic for mast cells, only SLF induces Ca²⁺ influx (Columbo *et al*., 1994 Rao *et al*., 1994). The effects on cell viability of two other signals known to mobilize Ca²⁺ in mast cells were thus investigated. Cross-linking of the high affinity receptor for IgE with antigen initiates a series of signaling events including PLC-γ activation and Ca²⁺ mobilization, which result in mast cell degranulation (Jouvin *et al*., 1995). The effect on cell viability of stimulating mast cells with Ag plus IgE in the presence of a Ca²⁺ influx blocker is shown in Figure 1E. As shown, cell death is induced when mast cells are stimulated with IgE plus antigen in the presence of econazole, whereas antigenic stimulation alone or econazole alone have no effect on cell viability. Ni²⁺ and ketotifen also combine with IgE receptor cross-linking to induce cell death (not shown).

Mast cells also respond to amphiphilic cationic peptides such as Substance P (Bueb *et al*., 1990 Mousli *et al*., 1990). These molecules directly stimulate heterotrimeric G-proteins resulting in PLC-β activation, Ca²⁺ mobilization and, in the presence of suboptimal levels of antigen plus IgE, mast cell degranulation. As shown in Figure 1F, cell death is also induced when mast cells are stimulated with Substance P in the presence of econazole. As with SLF, the degree of cell death in the presence of Ca²⁺ influx blocker increases with increasing concentrations of Ag or Substance P. Since these three signals (but not IL-3) all mobilize Ca²⁺ leading to Ca²⁺ influx, it is likely that Ca²⁺ mobilization is the required signal for the induction of cell death in combination with Ca²⁺ influx blockers.

Induction of cell death through the combination of an activating signal and a Ca²⁺ influx blocker in cells other than mast cells was also examined. 32D cells are c-kit negative, IL-3-dependent myelomonocytic cells that die apoptotically upon removal of factor (Baffy *et al*., 1993). Transfer and expression of c-kit in these cells makes them responsive to SLF *in vitro* and renders them tumorigenic in-vivo (Hu *et al*., 1995). As shown in Figure 2A, expression of c-kit makes these cells sensitive to induction of cell death by SLF and Ca²⁺ influx blocker.

Numerous factor independent tumors have been described that are transformed due to the constitutive activation or autocrine stimulation of growth factor receptors. An example of a factor independent tumor is the mastocytoma p815, which grows in the absence of added factor due to the presence of a mutated, constitutively activated c-kit receptor (Tsujimura *et al*., 1994). As shown in Figure 2B, Ca²⁺ influx blockers alone are sufficient to induce cell death in p815 cells in the absence of added SLF stimulus. This likely reflects the ligand-independent nature of signaling through the p815 c-kit receptor and therefore its ability to combine with a Ca²⁺ influx blocker to induce cell death. These results show that the induction of cell death by SLF plus Ca²⁺ influx blocker is not limited to mast cells, but can be observed in other cell types as well, particularly those with highly activated receptors.

### Experiment 2: BMMC and 32D-kit induced death is apoptotic.

Visual inspection of both mast cells and 32D-kit cells following treatment with SLF and a Ca²⁺ influx blocker revealed characteristics of apoptosis including nuclear condensation and membrane blebbing (Figure 3C). DNA content, which characteristically fragments and decreases during apoptosis, was also measured. As shown in Figures 4A and 4B and summarized in Table 1, treatment of 32D-kit cells with SLF or IL-3 alone for 18 hours did not generate a population of cells with subdiploid DNA content, whereas econazole plus SLF, but not IL-3, generated a large population of cells with subdiploid DNA (Figures 4C and 4D). 32D-kit cells are known to undergo apoptosis when they are deprived of growth factor.
After 18 hours of growth factor withdrawal, 32D-kit cells showed a modest proportion of cells with sub-diploid DNA content. This population is substantially increased after 24 hours of factor withdrawal (data not shown). Thus the apoptotic process induced by Ca²⁺ influx blocker plus SLF is more rapid than the apoptosis observed from factor withdrawal. Mast cells also exhibit sub-diploid DNA content following treatment with SLF plus Ca²⁺ influx blocker (Table 1), consistent with the induction of cell death by an apoptotic mechanism.

**Table 1.**

| **Sub G1 DNA Content of BMMCs or 32D-kit cells treated with econazole plus factor.** | | | |
|---|---|---|---|
| | | Sub G1 Content (%) | |
| | | no econazole | 8µM econazole |
| BMMC | | | |
| | IL-3 | 25.4 | 20.4 |
| | SLF | 6.8 | 66.8 |
| | Factor withdrawal | 6.18 | n.d. |
| 32D-kit | | | |
| | IL-3 | 1.6 | 4.8 |
| | SLF | 2.3 | 35 |
| | Factor withdrawal | 9.2 | n.d. |
| (1) BMMCs were incubated with 8µM econazole plus IL-3 (25% WEHI conditioned media), SLF (500ng/m.), or no factor for 24 hours, stained with propidium iodide, and analyzed for DNA content. | | | |
| (2) 32D-kit cells were similarly incubated for 18 hours before analysis. n.d.: not determined. | | | |

### Experiment 3: Protection of cells from SLF-Ca²⁺ influx blocker induced apoptosis by oleic acid.

The observation that Ca²⁺ mobilizing signals can combine with Ca²⁺ influx blockers to induce apoptosis suggests that this effect is mediated by activation of phospholipase C. In order to determine if PLC activation is important for cell death, the effect of oleic acid, which has been shown to inhibit PLC activation in response to epidermal growth factor (EGF) stimulation (Casabiell *et al*., 1993), on apoptosis induction was examined. This inhibitory effect, which does not alter EGF binding or EGF receptor tyrosine kinase activation, is only observed with *cis*-9-octadecenoic acid (oleic acid) but not with *trans*-9-octadecenoic acid (elaidic acid) (Casabiell *et al*., 1991). 32D-kit cells were incubated with econazole and SLF or IL-3 in the presence of either oleic or elaidic acid. As shown in Figure 5, 32D-kit cells treated with SLF and a Ca²⁺ channel blocker were rescued from cell death by oleic but not elaidic acid. The range of effectiveness of oleic acid is between 1-100µM which corresponds to concentrations required for PLC inhibition (Casabiell, Zugaza, 1993). This observation is consistent with a requirement for activation of phospholipase C in the induction of apoptosis in combination with Ca²⁺ influx blockers.

### Experiment 4: Protection of cells from SLF-Ca²⁺ influx blocker induced apoptosis by ionomycin.

Ca²⁺ influx following receptor activation is mediated by the opening of store-operated Ca²⁺ channels (also called lcrac) (Penner *et al*., 1993). It is likely that the lcrac channel is the target for inhibition since the efficacy with which the three compounds, ketotifen, econazole and Ni²⁺, induce cell death in combination with SLF correlates with their ability to inhibit lcrac (Franzius *et al*., 1994). It has also been observed that the voltage gated Ca²⁺ channel blockers verapamil and nifedipine are ineffective in inducing cell death when combined with SLF (not shown). This result suggests that the induction of cell death in combination with SLF is specific for non-voltage gated Ca²⁺ influx blockers.

Other non-specific effects have been reported for both econazole and ketotifen (Franzius, Hoth, 1994). In order to determine if blockade of Ca²⁺ influx is critical for induction of cell death, the effect of the calcium ionophore ionomycin on induction of apoptosis was examined. As shown in Figure 6A, ionomycin protects 32D-kit cells from SLF plus Ca²⁺ influx blocker-induced death in a concentration-dependent manner, with maximal protection at 10nM. Given the specificity of ionomycin for Ca²⁺ (Liu *et al*., 1978), these results indicate that it is the specific blockade of Ca²⁺ influx which is required for induction of apoptosis in combination with activation signals. Higher concentrations of ionomycin, which generate excessive levels of intracellular Ca²⁺, resulted in the restoration of cell death. This demonstrates that in the context of cell activation, both extremes of Ca²⁺ influx can induce cell death.

### Experiment 5: Effect of Bcl-2 on apoptosis induced in 32D-kit cells by SLF and plus Ca²⁺ influx blocker.

The Bcl-2 family of proteins have been strongly linked to protection of cells from apoptosis induced by a wide variety of agents. Expression of Bcl-2 is correlated with proliferating cells (Hockenbery *et al*., 1991; Veis *et al*., 1993), is negatively regulated by the tumor suppressor p53 (Miyashita *et al*., 1994), and overexpression of Bcl-2 protects 32D cells from apoptotic death following factor withdrawal (Nunez *et al*., 1990; Baffy, Miyashita *et al*., 1993). The effect of overexpression of Bcl-2 protein on the induction of apoptosis by SLF and Ca²⁺ influx blocker was thus examined. 32D-kit cells were infected with a retrovirus vector (Schwarze *et al*., 1995) containing the Bcl-2 gene, to generate a Bcl-2 overexpressing cell line (Figure 7A). The cells were tested for susceptibility to apoptosis and it was found that Bcl-2 overexpression protects 32D-kit cells from apoptosis induced by factor withdrawal (Figure 7B). However, as also shown in Figure 7B, overexpression of Bcl-2 in 32D-kit cells fails to protect these cells from induction of apoptosis by SLF and econazole. These observations led to the conclusion that induction of cell death by SLF plus blocker occurs in a Bcl-2 independent manner.

32Dkit-Bcl-2 cells were similarly protected from apoptosis by SLF plus blocker with low levels of ionomycin (Figure 6B), however, unlike the 32Dkit cells, they did not demonstrate significant cell death at higher levels of ionomycin. These results therefore show that for activated cells, death induced by Ca²⁺ blockade versus death induced by excessive levels of Ca²⁺ influx are divergent, at least at the level of Bcl-2 sensitivity.

### Experiment 6: Effect of an ICE protease inhibitor on Ca²⁺ influx blocker and SLF-induced cell death and cell death induced by factor withdrawal.

TNF-α and Fas ligand are two examples of apoptosis-inducing signals for which Bcl-2 overexpression fails to provide protection (Memon *et al*., 1995; Strasser *et al*., 1995). In these cases, apoptosis has been shown to be mediated by members of the interleukin-1β converting enzyme family of proteases. In order to determine if ICE-family proteases were involved in the induction of apoptosis by SLF and Ca²⁺ influx blocker, cells were co-incubated with the tetrapeptide-aldehyde ICE inhibitor YVAD-CHO (Mashima *et al*., 1995; Vasilakos *et al*., 1995). We found that YVAD-CHO protects 32D-kit cells from apoptosis induced by the topoisomerase inhibitor etoposide (not shown). It was also found that YVAD-CHO provides resistance to apoptosis induced by SLF plus econazole (Figure 8) but fails to protect 32D-kit cells from apoptosis induced by factor withdrawal (Figure 7B). It is therefore likely that apoptosis induced by the combination of a growth or activation signal with a Ca²⁺ influx blocker is mediated by an ICE-like protease.

### Experiment 7: Effect of Ca²⁺ blocker and EGF on human carcinoma cells.

Studies have demonstrated the involvement of growth factor receptors in breast cancer. For example, it has been found that over 45% of breast cancers were positive for the EGF^{R} (Fox *et al*., 1994; Franzius *et al*., 1994). erbB2, a growth factor receptor closely related to the EGF^{R} is overexpressed in 30-40% of all breast cancers (Slamon, *et al*., 1989). In order to determine if cell death can be induced in these cells with Ca²⁺ influx blockers plus activation signals, SK-BR3 cells were incubated with or without EGF and in presence of econazole. SK-BR3 cells (ATCC cat. # HTB-30) are derived from a human adenocarcinoma of the breast. They are tumorigenic in nude mice. These cells contain a mutated p53 gene (Elstner, *et al*., 1995), are estrogen-receptor negative, and express high levels of the erbB2 molecule (Li, *et al*., 1993) resulting in continuous activation of this receptor. As shown in Figure 9A, econazole induces equivalent levels of death in SK-BR3 cells in the presence or absence of EGF.

The MDA-MB-231 cell line is a highly anaplastic carcinoma of the breast. it too is tumorigenic in nude mice but expresses only low levels of erbB2. However it is FGF^{R} positive and is stimulated in an autocrine fashion by bFGF (el Yazidi *et al*., 1995). Experiments were carried out to determine the susceptibility of MDA-MB-231 cells to a Ca²⁺ influx blocker and the influence of oleic acid, ionomycin, and YVAD-CHO on induction of cell death by the blocker. The results are summarized together in Figure 9B. As indicated, MDA-MB-231 cells undergo apoptosis in response to exposure to the Ca²⁺ influx blocker econazole. Furthermore, the Ca²⁺ influx blocker-induced apoptotic mechanism of MDA-MB-231 appears to have the same characteristics as that of 32D-kit cells. Ionomycin, oleic acid and YVAD-CHO can protect MDA-MB-231 cells from death induced by the Ca²⁺ influx blocker.

MCF-7 cells (ATCC cat. # HTB-22) are derived from a human breast carcinoma. They are tumorigenic in nude mice, particularly when the mice are treated with estrogen (Benz, *et al*., 1993) or when the cells are encapsulated in matrigel (Noel, *et al*., 1995). These cells have been reported to be stimulated by EGF (Godden *et al*., 1992). In order to determine the susceptibility of these cells to cell death by Ca²⁺ influx blocker and activating signal, MCF-7 cells were incubated with and without 10ng/ml EGF, with various concentrations of econazole for 18 hours. As shown in Figure 10, MCF-7 cells are more sensitive to cell death from econazole in the presence of 10ng/ml EGF than in its absence. Taken together the experiments shown in Figures 9A, 9B and 10 indicate that some breast cancer cells will display increased sensitivity to blockers in the presence of an activating factor such as EGF.

### Experiment 8: Effect of Ca²⁺ influx blocker and SLF on 32D-kit leukemia cells in vivo

In order to determine the efficacy of the combination of a Ca²⁺ influx blocker with an activating signal *in vivo*, mice inoculated with factor-dependent and G418-resistant 32D-kit leukemia cells were treated with a combination of ketotifen and SLF. In one set of experiments, spleen cells were removed and assayed for the presence of leukemic cells by plating cells in agar in growth medium containing 20% WEHI-3 conditioned medium and 1mg/ml G418 to limit detection to the G418-resistant leukemic cells. In another set of experiments, spleens were assayed for the presence of all cells capable of forming colonies in agar in response to IL-3, to include leukemic as well as normal cells. As shown in Figure 11A, in each experiment, G418-resistant colony-forming cells can be detected in the inoculated animals. The levels found in the spleens of the inoculated animals varies from animal to animal, however the majority of the animals contain between 30 and 1,000 G418-resistant colonies. The arithmetic average colony count from the inoculated but untreated animals is 69,938, however one animal contained very high levels of leukemic cells. If this animal is not included, the arithmetic average colony count is 391. The geometric mean from this group is 813 colonies/spleen. The number of G418-resistant colonies detected in animals that were injected with SLF only tended to be higher than those not receiving the factor, varying between 65 and 31,000 with an arithmetic average of 7,399, and a geometric mean of 1,545 colonies/spleen. This increase in detectable colony number might be attributable to stimulation of leukemic cells by SLF. In mice receiving ketotifen alone, the number of detectable colonies varies between 114 and 27,645 with an arithmetic average of 5,502 and a geometric mean of 849. In animals treated with ketotifen and SLF, the number of detectable colonies varies from 8 to 537, with an arithmetic average of 131 and a geometric mean of 61 G418-resistant colonies/spleen. Analysis of this data using the one-tailed paired student's t-test (after log transformation) indicates that there is only a 6.2% probability that the untreated and treated groups are not different from each other. If the one animal in the untreated group that contained very high levels of leukemic cells is not included in this analysis, then the probability that the untreated and treated groups are not different from each other is 8.9%.

A similar comparison between the group of animals that received ketotifen alone versus those that received ketotifen plus SLF indicates that the probability that these groups are not different is 0.8% and is thus highly significant. This result indicates that while ketotifen alone has little activity on the leukemic cells *in vivo,* the combination of ketotifen and SLF does result in a significant decrease in leukemic cell number.

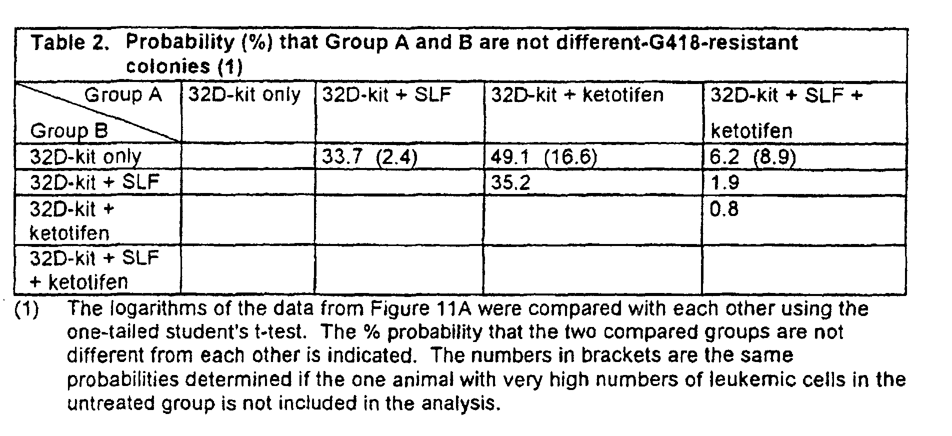

A similar analysis was performed for the total number of colony-forming cells in the spleens of these mice and the data are presented in Figure 11B. The arithmetic average colony number from mice not inoculated with leukemic cells is 857, with a geometric mean of 669. The arithmetic average colony count from the inoculated but untreated animals is 84,073, however one animal contained very high levels of leukemic cells. If this animal is not included, the arithmetic average colony count is 3,448. The geometric mean from this group is 4,573 colonies/spleen. The number of IL-3-dependent colonies detected in animals that were injected with SLF only tended to be higher than those not receiving the factor, with an arithmetic average of 18,212, and a geometric mean of 5,171 colonies/spleen. This increase in detectable colony number might be attributable to stimulation of leukemic cells by SLF. In mice receiving ketotifen alone, the arithmetic average is 7,715 with a geometric mean of 2,320. In animals treated with ketotifen and SLF, the arithmetic average was 793 with a geometric mean of 66 IL-3-dependent colonies/spleen. Analysis of these data using the one-tailed paired student's t-test (after log transformation) indicates that there is a 2% probability that the untreated and treated groups are not different from each other. If the one animal in the untreated group that contained very high levels of leukemic cells is not included in this analysis, then the probability that the untreated and treated groups are not different from each other is 5.1%. Similarly, a comparison of the groups treated with SLF only or ketotifen only versus those treated with ketotifen plus SLF together also indicates that there is a 2% or 1.6% probability that these groups are not different.

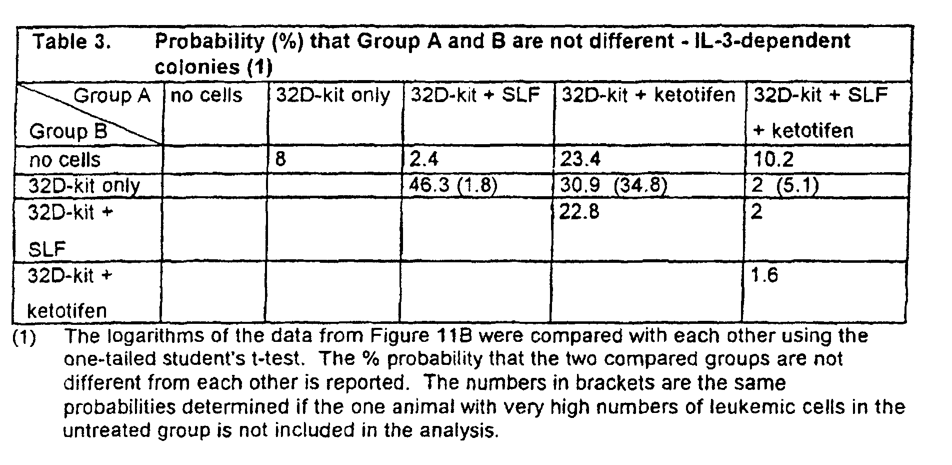

Another experiment was performed in which mice inoculated with 32D-kit cells were treated with ketotifen p.o. and SLF delivered subcutaneously. As shown in Figure 12, in this circumstance, the combination of SLF with ketotifen did not appear to have a significant effect on the number of leukemic cells in the spleens of the treated mice. This experiment indicates that the route of delivery of the activating factor and likely the relative timing of the two substances may affect the effectiveness of a particular therapeutic regimen.

The results of the above experiments describe a novel method for inducing apoptosis. This method involves the combination of blockade of Ca²⁺ influx into a cell, a receptor of which when activated under normal circumstances leads to Ca²⁺ influx, with a factor which activates the receptor. In particular embodiment, activation of phospholipase C is associated with the Ca²⁺ influx.

It has been shown, for example, that SLF, substance P, and IgE and antigen, but not IL-3, can combine with (non-voltage-gated) Ca²⁺ influx blockers to induce apoptosis in mast cells. This form of apoptosis does not necessarily require proliferative signals since both SLF and IL-3 are proliferative, whereas substance P and antigen-IgE are only weakly proliferative. Since increasing signal leads to increased apoptosis, this form of cell death will be most effective when Ca²⁺ influx blockers are combined with highly activated receptors. In support of this observation, the p815 mastocytoma, which has a constitutively activated c-kit receptor, is sensitive to Ca²⁺ influx blockers alone (Figure 2B). SK-BR3 and MDA-MB-231 cella are also susceptible to Ca²⁺ influx blockers alone. This also suggests that cells with similar mutations (or undergoing autocrine stimulation) may be susceptible to apoptosis by this class of drug.

The observation that oleic acid can reverse apoptosis induction by SLF plus blocker indicates a need for an activation signal that results in activation of phospholipase C in at least in some embodiments. Ionomycin can also protect cells from SLF plus blocker induced cell death, indicating that specific blockade of Ca²⁺ influx is required for apoptosis induction. Apoptosis induced by SLF plus blocker is insensitive to overexpression of Bcl-2, but can be reversed by an inhibitor of ICE proteases, suggesting a role in this apoptotic mechanism for ICE, or a member of the ICE family of proteases in at least some embodiments.

In that certain proliferating cells have highly activated receptors (Figure 9A) resulting in activation of PLC, this invention includes a method for diagnosing susceptibility of such cells to treatment with a Ca²⁺ influx blocker, or a blocker and factor which further activates receptors of the cells. Such a method includes:
obtaining a sample of diseased tissue;
determining whether the tissue contains a level of PLC elevated in comparison to normal tissue;
if the tissue contains an elevated level of PLC, then determining whether the PLC is activated.
If such an elevated level of activated PLC is found in the tissue, then the cells are likely to be treatable, i.e., their death induced, proliferation diminished, etc., by exposure of the cells to a Ca²⁺ influx blocker or a Ca²⁺ influx blocker and a factor which further activates the receptors which produce the signal that leads to elevated levels of activated PLC.

Anti-PLC-specific anti-sera has been used to show that breast carcinomas have elevated levels of the enzyme compared to normal controls. Immunoprecipitation with anti-phosphotyrosine antibodies, followed by Western blotting with anti-PLC antibodies can be used to determine levels of activated PLC because PLC that is tyrosine-phosphorylated is likely to be activated (Arteaga *et al.* 1991).

PLC enzymatic activity can be assayed directly (Huang *et al*., 1995). Briefly, cells to be assayed are lysed, PLC immunoprecipitated using PLC-specific antisera (available, for example, from Upstate Biotechnology Inc, Lake Placid, New York) and the enzyme assayed using [³H]-PIP₂ (from New England Nuclear, Boston, Massachusetts) as a substrate in liposomes (90% DMPM, 10% PIP₂) in a buffer containing 0.3mM total lipid in 50 mM tris (pH 8), 0.1 mM CaCl₂. Reactions are terminated by the addition of 0.2M CaCl₂ in 1M HCI. Precipitated vesicles are collected on filter plates (such as Millipore 96-well hydrophobic MultiScreen DP plates) and the inositol phosphate product of the reaction, which is present in the flow through, is quantitated by scintillation counting.

The observation that ionomycin can both protect against or induce cell death indicates that both extremes of Ca²⁺ influx can lead to apoptosis. In both cases however, cell activation appears to be required since only minimal cell death was observed in the absence of SLF. Bcl-2 overexpression protected cells only from death induced by high concentrations of ionomycin, suggesting that mechanisms underlying cell death at the two extremes of Ca²⁺ influx are not identical, at least at the level of sensitivity to Bcl-2. Thus, at high concentrations of ionomycin, the cells may be subjected to a form of "Ca²⁺ overload" and certain cells exposed to this form of toxicity are known to be protected by Bcl-2 (Strasser *et al*., 1991; Reed, 1994). It has been postulated that a possible mediator of high Ca²⁺ death may be the Ca²⁺-dependent phosphatase calcineurin, which has been shown to potentiate apoptosis in T cells (Shibasaki *et al*., 1995) and B cells (Bonnefoy *et al*., 1994).

Results described herein (Figures 7B and 8) indicate that Bcl-2 and ICE are involved in regulation of different apoptosis mechanisms. While cells are protected by Bcl-2 (but not the ICE inhibitor) from apoptosis resulting from factor withdrawal, ICE inhibitor (but not Bcl-2) protects against the combination of Ca²⁺ influx blocker and SLF. Experiments with other apoptosis-inducing signals are also consistent with these observations. (Cuende, *et al*., 1993; Memon *et al*., 1995; Strasser *et al*., 1995; Vasilakos *et al*., 1995; Parijs *et al*., 1996). It has also recently been demonstrated that Fas activation inhibits anti-CD3-mediated Ca²⁺ influx in T cells without affecting Ca²⁺ release from internal stores (Kovacs *et al*., 1995).

Ketotifen is a well known anti-allergy and anti-asthmatic drug that exhibits both histamine H1 receptor antagonism and inhibition of Ca²⁺ influx (Martinet *al*., 1978; Grant *et al*., 1990). Given the involvement of Ca²⁺ influx in mast cell degranulation, inhibition of this process is one accepted mechanism for its anti-inflammatory effects. The results described herein thus suggest that a Ca²⁺ influx blocker, particularly ketotifen, can be co-administered with an appropriate activation signal for treatment of allergy symptoms. Ca²⁺ influx blockers have been proposed as anti-proliferative agents (Riccardi, 1987; Felder *et al*., 1991; Kohn et *al*., 1992; Estacion *et al*., 1993; Petrasch *et al*., 1993; Kondo *et al*., 1994; Buckley *et al*., 1995; De Vries *et al*., 1995). The results described herein suggest that these compounds would be more effective when coupled with a strong proliferative or activating signal. Therefore the combination of Ca²⁺ influx blockers with proliferative or activating signals may have potent, but specific, anti-proliferative or anti-inflammatory properties.

The inventors do not wish to be bound by any theory explaining the precise mechanism by which Ca²⁺ influx into cells is caused. The results presented herein do, however, establish a connection between the induction of cell death upon administration to the cell of a combination of a Ca²⁺ influx blocker and a factor which binds to a receptor of such a cell to cause under normal conditions activation of the receptor so as to cause Ca²⁺ influx.

Such factor can be a natural ligand of the receptor, i.e., a ligand that acts upon the receptor in nature, an agonistic antibody etc. At least in some cases, the influx involves activation of PLC. In may cases, activation involves stimulation of the cell wherein "stimulation" includes proliferation, activation, and/or survival of the cell.

The results shown in Figure 9A indicate, that in the case of constitutively activated cells, in which the receptor is normally activated by a ligand followed by Ca²⁺ influx, administration of a Ca²⁺ influx blocker alone can induce cell death. Thus also a Ca²⁺ influx blocker could be administered to induce death in cells having a constitutively activated receptor, in which activation of the receptor promotes influx of Ca²⁺ into the cell.

It is also possible for cells having receptors which are activated in response to a factor produced by the cell itself, to be treated with a Ca²⁺ blocker to induce cell death.

With respect to particular known factors, SLF for example, variants of SLF, the c-kit receptor ligand, can also be effective in inducing cell death. For example, agonistic antibodies to the c-kit receptor could be developed according to procedures known to those skilled in the art. Other small molecules capable of cross-linking receptors and inducing the Ca²⁺ influx might also be effective. SLF itself might be modified by mutations to stabilize the molecule and thereby extend its half-life in a mammlian body. Alternatively, or additionally, SLF could be modified by conjugation with polyethylene glycol (PEG) in order to extend its longevity in use.

In place of IgE and antigen thereto in the case of mast cells, for example, an antibody to the cellular IgE receptor itself could be used. It is likely that an antibody capable of crosslinking IgE receptors would be most likely to cause Ca²⁺ influx to occur.

In the case of those suffering from allergies, immunoglobulins specific to the allergen of the class IgE are produced by B cells that have been stimulated by allergen-specific T cells to produce this particular isotype. IgE antibodies are secreted by B cells into the extracellular space where they can bind to specific receptors called FcεRI found on the surface of mast cells or basophils. Should these mast cells or basophils encounter a polyvalent allergen that is recognized by their surface bound IgE, the IgE/receptors can be cross-linked and activated, causing the release of one or more pre-formed mediators such as histamine, and the eventual synthesis and release of other inflammatory molecules such as leukotrienes, prostaglandins and cytokines. This complex response therefore involves a number of different cell types and molecules, all of which are possible targets in modulating the allergic response. This includes B cells producing allergen-specific IgE, T cells expressing T cell receptors specific for the allergen, and mast cells and basophils, with allergen-specific IgE on their surface. Since the signals transmitted through these receptors all mobilize Ca²⁺, they will be able to combine with Ca²⁺ influx blockers to induce cell death, as has been shown for IgE plus antigen on mast cells. For B cells expressing membrane IgE, specific anti-IgE compounds will cross-link these molecules, resulting in the transmission of a signal. Such compounds may be antibodies, such as described by Chang in United States Patent Nos. and 5,422,258 and 5,428,133 or Rupp *et al*. in United States Patent No. 4,940,782, for example.

In addition, fragments of these antibodies such as (Fab)₂ reagents or other compounds that recognize and cross-link surface IgE on B cells may be employed. When B cells are being targeted specifically, it would be desirable to use reagents that preferentially recognize IgE on the surface of B cells, but not IgE as it is bound on mast cells or basophils.

In situations where it is desirable to specifically eliminate B cells producing antibodies that recognize a particular antigen, the antigen itself, if polyvalent and given in sufficient quantity, may provide a sufficient signal in combination with a Ca²⁻ influx blocker such that the B cells producing the antibody will be killed. Alternatively, anti-idiotypic antibodies, that recognize the variable regions of the antibody being produced by the B cell may also be used. Potential targets for such a treatment would include B cells producing antibodies to an autoantigen, resulting in an autoimmune disease.

There are, of course, other cellular receptors ligand-binding of which results in Ca²⁺ influx into the cell bearing the receptor. Growth factor receptor agonists within the scope of this invention include TGF-α (transforming growth factor), EGF, heregulin, and other agonists of erbB1, 2, 3, & 4, PDGF A and B (platelet-derived growth factor), SLF, FLT-3 (fms-like tyrosin kinase-3) ligand, basic and acidic FGFs (fibroblast growth factors) (a family of related growth factors), enothelin, NGF (nerve growth factor), VEGF (vascular endothelial growth factor), HGF (SF) (hepatic growth factor; scatter factor). Agonists to multi-chain immune recognition receptors include agonists to the antigen receptor on B cells (i.e, specific antigens recognized by B cells) including antibodies that cross-link surface immunoglobulin, T cell receptor agonists such as antibodies recognizing particular TCRs, anti-CD3 antibodies or other agonistic aggregated antibodies and other reagents that activate FcγRII and FcγRIII, and agonists to the FceRI receptor on mast cells and basophils. Agonists of G-protein-linked receptors are also within the scope of the teachings of this invention: bombesin, gastrin, gastrin releasing peptide, bradykinin, carbachol and other muscarinic receptor agonists, CCK-8, vasopressin, neurokinins such as substance P, purinergic receptor agonists including ATP and adenosine, 1,25-dihydroxyvitamin D₃ There are also many neurotransmitters that stimulate Ca²⁺ influx. Cell death can be shown to occur as the result of administration to such cells of a Ca²⁺ influx blocker and ligand using experiments similar to those disclosed herein. Variants of such ligands and agonistic receptor antibodies can also be developed for use and tested using experiments similar to those described above in which cell death is measured in response to administration of such a variant in combination with a Ca²⁺ influx blocker.

Calcium influx blockers in addition to those described in connection with the preferred embodiments include tenidap, CAl, Cd²⁺, Co²⁺, La³⁺, Mn²⁺, SKF-96365 and cromolyn, although there are many more than these known to those skilled in the art (Lewis, *et al*., 1995).

In any particular case, it may be found that a combination of receptor-binding agents administered in combination with one or more Ca²⁺ influx blockers turns out to be most effective in eliciting the desired cellular response.

As indicated by the experiments disclosed herein, it is the receptor and the normal response of the cell to signals generated on appropriate receptor binding which is important in determining whether cell death can be induced by administration of a receptor ligand (or other agonistic agent) and a Ca²⁺ influx blocker. Nonetheless, it is known that cell types beyond those used in the experiments disclosed herein have such receptors expressed, at least during some point of their life cycle, which will produce the normal cellular response required for application of the invention disclosed herein. Examples of other cell types known to express the c-kit receptor and be responsive to SLF are haematopoietic progenitor cells, melanocytes and germ cells. Examples of cell types which can express receptors which bind other ligands, which binding normally results in the requisite response include breast cancer cells expressing EGF and FGF receptors and colon carcinoma cells expressing PDGF receptors.

Such cells displaying tumorigenic, hyperplastic or highly active states may be treated by administration of an appropriate binding factor and Ca²⁺ influx blocker according to the present invention. It will thus be possible to treat a person suffering from cancer, mastocytosis, autoimmune inflammatory diseases or an allergy, for instance.

A c-kit receptor has been found to be overexpressed on leukemia cells (Muroi *et al*., 1995) and to be found in high proportion of small cell lung cancers (Hida *et al*., 1994), tumors of the female genital tract (Inoue *et al*., 1994), and may be involved in mast cell infiltration of neurofibromas (Hirota *et al*., 1993). Such sites are thus prime candidates for the present invention.

In instances in which cells to be targeted according to the present invention are constitutively activated, (Figure 9A) it might not be necessary to include an agent to stimulate the signal generated by receptor binding, as signal is already being generated. In such an instance, a Ca²⁺ influx blocker can be administered without the accompanying receptor-stimulating agent. Certain tumor cells are known to possess such activity and hence could be treated by this method. A c-kit activating mutation has been found in mastocytomas of human and rodent origin (Kanakura *et al*., 1994; Kitayama *et al*., 1995). This is not to say that it would not be preferred in some circumstances to potentiate the signal by administration of a suitable factor along with a Ca²⁺ blocker.

A starting step in determining whether a particular cell type would be susceptible to treatment according to the present invention would be to determine whether ligand binding of a receptor expressed on the cell membrane causes an increase in the concentration of intracellular Ca²⁺. Administration of various concentrations of the ligand and a Ca²⁺ influx blocker cell cultures, in analogy to experiments described for the cells of the embodiments disclosed herein, could then be carried out to determine levels at which cell death is induced.

For particular indications, the appropriate dosage will vary depending upon, for example, the host, mode of administration and the nature and severity of the condition to be treated, etc. Satisfactory results are indicated for a receptor-binding agent to be obtained at daily dosages of, say, from about 0.1 mg per kg of animal body weight to about 1 mg per kg of body weight although this may vary depending upon the particular agent or the subject or the condition being treated. Further, it may be advantageous for the daily dosage to be divided into 2, 3, 4 or more doses.

If the agent is a polypeptide such as a growth factor, administration could be by intradermal, intramuscular or intravenous injection, for example.

Pharmaceutical compositions containing a receptor binding compound of the present invention would include at least one pharmaceutically acceptable carrier, diluent, excipient, lubricant, buffer, antibacterial, bulking agent, anti-oxidants or the like, the composition being manufactured in a conventional manner by mixing with the pharmaceutically acceptable carrier or diluent, etc. Unit dosage forms contain, for example, from about 0.025 mg to about 50 mg of the compound.

Known Ca²⁺ influx blockers could be prepared and administered according to known procedures, provided the agent reached the target cells as desired.

In order to target a specific cell, say for example, a B cell which produces a particular IgE, an agonistic antibody to the surface portion of the IgE could be generated. A suitable Ca²⁺ influx blocker (or blockers) could be suitably linked (for example, covalently linked) to the antibody such that binding of the antibody to the surface IgE would bring the blocker into contact with the particular cell on which it is desired to act.

### References

Particulars of references cited above are given below. All of the listed references are incorporated herein by reference.
Arteaga, C.L., Johnson, M.D., Todderud, G., Coffey, R.J., Carpenter, G., Page, D.L. (1991) Elevated content of the tyrosine kinase substrate phospholipase C-gamma 1 in primary human breast carcinomas. *Proc. Natl. Acad. Sci. U.S.A.,* **88,** 10435-9.
Ashwell, J.D., Cunningham, R.E., Noguchi, P.D. & Hernandez, D. (1987) Cell growth cycle block of T cell hybridomas upon activation with antigen. *J. Exp. Med*., **165**, 173-194.
Baffy, G., Miyashita, T., Williamson, J.R. & Reed, J.C. (1993) Apoptosis induced by withdrawal of interleukin-3 (IL-3) from an IL-3-dependent hematopoietic cell line is associated with repartitioning of intracellular calcium and is blocked by enforced Bcl-2 oncoprotein production. *J. Biol. Chem*., **268**, 6511-9.
Bargou, R. C., P. T. Daniel, M. Y. Mapara, K. Bommert. C. Wagener, B. Kallinich, H. D. Royer and B. Dorken. (1995) Expression of the bcl-2 gene family in normal and malignant breast tissue: low bax-alpha expression in tumor cells correlates with resistance towards apoptosis. *Int. J. Cancer.* **60,** 854-9.
Benz, C. C., G. K. Scott, J. C. Sarup, R. M. Johnson, D. Tripathy, E. Coronado, H. M. Shepard and C. K. Osborne, (1993) Estrogen-dependent, tamoxifen-resistant tumorigenic growth of MCF-7 cells transfected with HER2/neu. *Breast Cancer Res. & Trtmnt*. **24**, 85-95.
Benzaquen, L. R., C Brugnara, H. R. Byers, S. Gattoni-Celli and J. A. Halpern (1995) Clotrimazole inhibits cell proliferation *in vitro* and *in vivo. Nature Medicine* **1**, 534-40.
Berger, S.A., Mak, T.W. & Paige, C.J. (1994) Leukocyte common antigen (CD45) is required for immunoglobulin E-mediated degranulation of mast cells. *J. Exp. Med*., **180**, 471-6.
Bonnefoy, B.N., Genestier, L., Flacher, M. & Revillard, J.P. (1994) The phosphoprotein phosphatase calcineurin controls calcium-dependent apoptosis in B cell lines. *Eur. J. Immunol*., **24**, 325-9.
Brown, D.M., Warner, G.L., Ales-Martinez, J.E., Scott, D.W. & Phipps, R.P. (1992) Prostaglandin E2 induces apoptosis in immature normal and malignant B lymphocytes. *Clin. Immunol.I mmunopathol*., **63**, 221-9.
Buckley, N.E., Su, Y., Milstien, S. & Spiegel, S. (1995) The role of calcium influx in cellular proliferation induced by interaction of endogenous ganglioside GM1 with the B subunit of cholera toxin. *Biochim. Biophys. Acta*, **1256**, 275-83.
Bueb, J.L., Mousli, M., Bronner, C., Rouot, B. & Landry, Y. (1990) Activation of Gi-like proteins, a receptor-independent effect of kinins in mast cells. *Molec. Pharmacol.*, **38**, 816-22.
Casabiell, X., Pandiella, A. & Casanueva, F. (1991) Regulation of epidermal-growth-factor-receptor signal transduction by cis-unsaturated fatty acids. *Biochem. J*., **278**, 679-87.
Casabiell, X., Zugaza, J.L., Pombo, C.M., Pandiella, A. & Casanueva, F.F. (1993) Oleic acid blocks epidermal growth factor-activated early intracellular signals without altering the ensuing mitogenic response. *Exp*. *Cell Res.*, **205**, 365-73.
Chang, T-w. Chimeric anti-human IgE-monoclonal antibody which binds to secreted IgE and membrane-bound IgE expressed by IgE-expressing B cells but not to IgE bound to FC receptors. United States Patent No. 5,428,133 issued June 27, 1995.
Chang, T-w. Methods for producing high affinity anti-human IgE-monoclonal antibodies which bind to IgE on IgE-bearing B cells but not basophils. United States Patent No. 5,422,258 issued June 6, 1995.
Chinnaiyan A.M., Orth, K., O'Rourke, K., Duan, H., Poirier, G.G. & Dixit, V.M. (1996) Molecular ordering of the cell death pathway. *J. Biol. Chem*. **271**, 4573-6.
Columbo, M., Botana, L.M., Horowitz, E.M., Lichtenstein, L.M. & MacGlashan, D.J. (1994) Studies of the intracellular Ca2+ levels in human adult skin mast cells activated by the ligand for the human c-kit receptor and anti-IgE. *Biochem. Pharmacol*., **47**, 2137-45.
Cuende, E., Ales-Martinez, J.E., Ding, L., Gonzalez-Garcia, M., Martinez-A, C. & Nunez, G. (1993) Programmed cell death by bcl-2-dependent and independent mechanisms in B lymphoma cells. *Embo J.,* **12**, 1555-60.
Denyer et al. (1985) The effects of econazole on the growth of murine myeloma cells and its use in hybridoma production. *Developments in Biological Standardization*, **60**, 503-8.
De Vries, G.W., McLaughlin, A., Wenzel, M.B., Perez, J., Harcourt, D., Lee, G., Garst, M. & Wheeler, L.A. (1995) The antiinflammatory activity of topically applied novel calcium-channel antagonists. *Inflammation*, **19**, 261-75.
Dowd, D.R (1995) Calcium regulation of apoptosis. *Adv. Second Mess.* & *Phospho. Prot.* Res., **30,** 255-80.
el Yazidi, I. and Y. Boilly Marer. (1995) Production of acidic and basic fibrobiast growth factor by the hormone-independent breast cancer cell line MDA-MB-231. *Anticancer Res.* **15,** 783-90.
Elstner, E., I. M. Linker, J. Said, T. Umiel, V. S. de, I. P. Shintaku, D. Heber, L. Binderup, M. Uskokovic and H. P. Koeffler. (1995) 20-epi-vitamin D3 analogues: a novel class of potent inhibitors of proliferation and inducers of differentiation of human breast cancer cell lines. *Cancer Res.* **55,** 2822-30.
Enari, M., Hug, H. & Nagata, S. (1995) Involvement of an ICE-like protease in Fas-mediated apoptosis. *Nature,* **375**, 78-81.
Estacion, M. & Mordan, L.J. (1993) Competence induction by PDGF requires sustained calcium influx by a mechanism distinct from storage-dependent calcium influx. *Cell calcium*, **14**, 439-54.
Ethier, S. P. (1995) Growth factor synthesis and human breast cancer progression. [Review]. *J. Natl. Cancer Inst.* **87**, 964-73.
Felder, C.C., Ma, A.L., Liotta, L.A. & Kohn, E.C. (1991) The antiproliferative and antimetastatic compound L651582 inhibits muscarinic acetylcholine receptor-stimulated calcium influx and arachidonic acid release. *J. Pharmacol. Exp. Therapeut*., **257**, 967-71.
Fox, S. B., K. Smith, J. Hollyer, M. Greenall, D. Hastrich and A. L. Harris. (1994) The epidermal growth factor receptor as a prognostic marker: results of 370 patients and review of 3009 patients. *Breast Cancer Res. & Trtmnt*. **29**, 41-9.
Franzius, D., Hoth, M. & Penner, R. (1994) Non-specific effects of calcium entry antagonists in mast cells. *Pflugers Archiv* /*Eur.J. Physiol*., **428**, 433-8.
Fry, D. W. and J. M. Nelson. (1995) Inhibition of basic fibroblast growth factor-mediated tyrosine phosphorylation and protein synthesis by PD 145709, a member of the 2-thioindole class of tyrosine kinase inhibitors. *Anti Cancer Drug Design.* **10**, 607-22.
Godden, J., R. Leake and D. J. Kerr. (1992) The response of breast cancer cells to steroid and peptide growth factors. *Anticancer Res*. **12**, 1683-8.
Grant, S.M., Goa, K.L., Fitton, A. & Sorkin, E.M. (1990) Ketotifen. A review of its pharmacodynamic and pharmacokinetic properties, and therapeutic use in asthma and allergic disorders. *Drugs,* **40**, 412-48.
Gushchin et al. (1985) Ketotifen effect on secretion of histamine from basophils and on proliferative response of mononuclear cells of human peripheral blood. *Allergologia et Immunopathologia*, **13**, 111-21.
Hida, T., R. Ueda, Y. Sekido, K. Hibi, R. Matsuda, Y. Ariyoshi, T. Sugiura, T. Takahashi and T. Takahashi. (1994) Ectopic expression of c-kit in small-cell lung cancer. *Int. J. Cancer. Suppl*. **8**, 108-9.
Hines, S. J., C. Organ, M. J. Kornstein and G. W. Krystal. (1995) Coexpression of the c-kit and stem cell factor genes in breast carcinomas. *Cell Growth & Diff*. **6**, 769-79.
Hirota, S., S. Nomura, H. Asada, A. Ito, E. Morii and Y. Kitamura. (1993) Possible involvement of c-kit receptor and its ligand in increase of mast cells in neurofibroma tissues. *Archives of Pathology & Laboratory Medicine*. **117**, 996-9.
Hockenbery, D.M., Zutter, M., Hickey, W., Nahm, M. & Korsmeyer, S.J. (1991) BCL2 protein is topographically restricted in tissues characterized by apoptotic cell death. *Proc. Natl. Acad. Sci. U.S.A.,* **88**, 6961-5.
Honn, K. V., J. D. Taylor and J. M. Onoda. Method and composition for the treatment of tumors by administering a platinum coordination compound and a calcium channel blocker compound of the dihydropyridine class. United States Patent No. 4,906,646 issued March 6, 1990.
Horak, E., K. Smith, L. Bromley, S. LeJeune, M. Greenall, D. Lane and A. L. Harris. (1991) Mutant p53, EGF receptor and c-erbB-2 expression in human breast cancer. *Oncogene*. **6**, 2277-84.
Hoth, M. & Penner, R. (1992) Depletion of intracellular calcium stores activates a calcium current in mast cells. *Nature,* **355**, 353-6.
Hoth, M. & Penner, R. (1993) Calcium release-activated calcium current in rat mast cells. J. *Physiol*., **465**, 359-86.
Hu, Q., Trevisan, M., Xu, Y., Dong, W., Berger, S.A., Lyman, S.D. & Minden, M.D. (1995) c-KIT expression enhances the leukemogenic potential of 32D cells. *J. Clin. Invest*., **95**, 2530-8.
Huang, P.S, Davis, L., Huber, H., Goodhart, P.J., Wgrzyn, R.E., Oliff, A., & Heimbrook, D.C. (1995) An SH3 domain is required for the mitogenic activity of microinjected phospholipase C-gamma 1. *FEBS Letters*, **358,** 287-92.
Inoue, M., S. Kyo, M. Fujita, T. Enomoto and G. Kondoh. (1994) Coexpression of the c-kit receptor and the stem cell factor in gynecological tumors. *Cancer Res.* **54,** 3049-53.
Jones, L.A., Chin, L.T., Longo, D.L. & Kruisbeek, A.M. (1990) Peripheral clonal elimination of functional T cells. *Science*, **250**, 1726-9.
Jouvin, M.H., Numerof, R.P. & Kinet, J.P. (1995) Signal transduction through the conserved motifs of the high affinity IgE receptor Fc epsilon RI. *Sem. Immunol.*, **7**, 29-35.
Kanakura, Y., T. Furitsu, T. Tsujimura, J. H. Butterfield, L. K. Ashman, H. Ikeda, H. Kitayama, Y. Kanayama, Y. Matsuzawa and Y. Kitamura. (1994) Activating mutations of the c-kit proto-oncogene in a human mast cell leukemia cell line. *Leuk* **8** Suppl 1, 18-22.
Kitayama, H., Y. Kanakura, T. Furitsu, T. Tsujimura, K. Oritani, H. lkeda, H. Sugahara, H. Mitsui, Y. Kanayama, Y. Kitamura and a. I. et. (1995) Constitutively activating mutations of c-kit receptor tyrosine kinase confer factor-independent growth and tumorigenicity of factor-dependent hematopoietic cell lines. *Blood.* **85**, 790-8.
Kizaki, H., Tadakuma, T., Odaka, C., Muramatsu, J. & lshimura, Y. (1989) Activation of a suicide process of thymocytes through DNA fragmentation by calcium ionophores and phorbol esters. *J. Immunol*., **143**, 1790-4.
Kohn, E.C., Sandeen, M.A. & Liotta, L.A. (1992) *In vivo* efficacy of a novel inhibitor of selected signal transduction pathways including calcium, arachidonate, and inositol phosphates. *Cancer Res*., **52**, 3208-12.
Kohn, E. C., W. Jacobs, Y. S. Kim, R. Alessandro, S.W. Stetier and L. A. Liotta. (1994) Calcium influx modulates expression of matrix metalloproteinase-2 (72-kDa type IV collagenase, gelatinase A). J. *Biol. Chem.* **268**, 21505-11.
Kohn E. C., Felder, C. C., Jacobs, W., Holmes, K. A., Day, A., Freer R., and Liotta, L. A. (1994a) Structure-function analysis of signal and growth inhibition by carboxyamidotrizole, CAI. *Cancer Res*., **54**, 935-42.
Kondo, N., Fukutomi, O., Kameyama, T., Nishida, T., Li, G.P., Agata, H., Shinbara, M., Shinoda, S., Yano, M. & Orii, T. (1994b) Suppression of proliferative responses of lymphocytes to food antigens by an anti-allergic drug, ketotifen fumarate, in patients with food-sensitive atopic dermatitis. *Int. Arch. Allergy & Immunol.*, **103**, 234-8.
Kopreski, M. S., A. Lipton, H. A. Harvey and R. Kumar. (1996) Growth inhibition of breast cancer cell lines by combinations of anti-P185HER2 monoclonal antibody and cytokines. *Anticancer Res*. **16**, 433-6.
Korsmeyer. S.J. (1995) Regulators of cell death. *Trends Genet.*, **11**, 101-5.
Kovacs, B. & Tsokos, G.C. (1995) Cross-linking of the Fas/Apo-1 antigen suppresses the CD3-mediated signal transduction events in human T lymphocytes. *J. Immunol.*, **155,** 5543-9.
Krajewski, S., C. Blomqvist, K. Franssila, M. Krajewska, V. M. Wasenius, E. Niskanen, S. Nordling and J. C. Reed. (1995) Reduced expression of proapoptotic gene BAX is associated with poor response rates to combination chemotherapy and shorter survival in women with metastatic breast adenocarcinoma. *Cancer Res*. **55**, 4471-8.
Lampe. P.A., Cornbrooks, E.B., Juhasz, A., Johnson, E.J. & Franklin, J.L. (1995) Suppression of programmed neuronal death by a thapsigargin-induced Ca2+ influx. *J. Neurobiol*., **26**, 205-12.
Li, B. D., K. D. Bauer, W. P. Carney and R. B. Duda. (1993) Detection of c-erbB-2 oncoprotein expression in breast tissue by multiparameter flow cytometry. *J. Surg. Res.* **54**, 179-88.
Lee, E. Y. (1995) Tumor suppressor genes and their alterations in breast cancer. *Sem. Cancer Biol*. **6**, 119-25.
LeJeune, S., R. Leek, E. Horak, G. Plowman, M. Greenall and A. L. Harris. (1993) Amphiregulin, epidermal growth factor receptor, and estrogen receptor expression in human primary breast cancer. Cancer *Res.* **53**, 3597-602.
Levitzki, A. and A. Gazit. (1995) Tyrosine kinase inhibition: an approach to drug development. [Review]. *Science.* **267**, 1782-8
Lewis, R.S. & Cahalan, M.D. (1995) Potassium and calcium channels in lymphocytes. *Annu. Rev. Immunol*., **13**, 623-53.
Liu, C. & Hermann, T.E. (1978) Characterization of ionomycin as a calcium ionophore. *J. Biol. Chem*., **253**, 5892-4.
Lu, Q. L., P. Abel, C. S. Foster and E. N. Lalani. (1996) bcl-2: role in epithelial differentiation and oncogenesis. [Review] *Hum. Pathol*. **27**, 102-10.
Lupu, R., R. B. Dickson and M. E. Lippman. (1992) The role of erbB-2 and its ligands in growth control of malignant breast epithelium. [Review] J. *Ster. Biochem. & Molec. Biol.* **43**, 229-36.
Martin, U. & Romer, D. (1978) The pharmacological properties of a new, orally active antianaphylactic compound: Ketotifen, a Benzocycloheptathiophene. *Arzneim*.-*Forsch.lDrug Res*., **28**, 770-82.
Mashima, T., Naito, M., Fujita, N., Noguchi, K. & Tsuruo, T. (1995) Identification of actin as a substrate of ICE and an ICE-like protease and involvement of an ICE-like protease but not ICE in VP-16-induced U937 apoptosis. *Biocem*. *Biophys. Res. Commun*., **217**, 1185-92.
McConkey, D.J., Harzell, P., Amador-Perez, J.F., Orrenius, S. & Jondal, M. (1989) Calcium-dependent killing of immature thymocytes by stimulation via the CD3/T cell receptor complex. *J. Immunol*., **143**, 1801.
Memon, S.A., Moreno, M.B., Petrak, D. & Zacharchuk, C.M. (1995) Bcl-2 Blocks Glucocorticoid- but not Fas- or Activation-Induced Apoptosis in a T Cell Hybridoma. *J. Immunol*., **155,** 4644-52.
Mercep, M., Noguchi, P.D. & Ashwell, J.D. (1989) The cell cycle block and lysis of an activated T cell hybridoma are distinct processes with different Ca2+ requirements and sensitivity to Cyclosporine A. *J. Immunol*., **142**, 4085-92.
Miura, M., Friedlander, R.M. & Yuan, J. (1995) Tumor necrosis factor-induced apoptosis is mediated by a CrmA-sensitive cell death pathway. *Proc. Natl. Acad. Sci. U.S.A.*, **92**, 8318-22.
Miyashita, T., Krajewski, S., Krajewska, M., Wang, H.G., Lin, H.K., Liebermann, D.A., Hoffman, B. & Reed, J.C. (1994) Tumor suppressor p53 is a regulator of bcl-2 and bax gene expression in vitro and in vivo. *Oncogene*, **9**, 1799-805.
Mousli, M., Bueb, J.L., Bronner, C., Rouot, B. & Landry, Y. (1990) G protein activation: a receptor-independent mode of action for cationic amphiphitic neuropeptides and venom peptides. *Trends Pharmacol. Sci.,* **11**, 358-62.
Muroi, K., M. Nakamura, Y. Amemiya, T. Suda and Y Miura. (1995) Expression of c-kit receptor (CD117) and CD34 in leukemic cells. [Review]. *Leukemia* & *Lymphoma.* ***16***, *297-305*.
Noel, A., V. Borcy, M. Bracke, C. Gilles, J. Bernard, P. Birembaut, M. Mareel and J. M. Foidart. (1995) Heterotransplantation of primary and established human tumour cells in nude mice. *Anticancer Res*. **15**, 1-7.
Nicotera, P., Zhivotovsky, B. & Orrenius, S. (1994) Nuclear calcium transport and the role of calcium in apoptosis. *Cell Calcium*, **16**, 279-88.
Nunez, G., London, L., Hockenbery, D., Alexander, M., McKearn, J.P. & Korsmeyer, S.J. (1990) Deregulated Bcl-2 gene expression selectively prolongs survival of growth factor-deprived hemopoietic cell lines. J. *Immunol*., **144**, 3602-10.
Oltvai, Z.N., Milliman, C.L. & Kormeyer, S.J. (1993) Bcl-2 heterodimerizes in vivo with a conserved homolog, Bax, that accelerates programmed cell death. *Cell*, **74**, 609-619.
Orrenius, S., Burkitt, M.J., Kass, G.E.N., Dypbukt, J.M. & Nicotera, P. (1992) Calcium ions and oxidative cell injury. *Ann. Neurol*., **32**, S33-S42.
Parijs, L.V., Ibraghimov, A. & Abbas, A.K. (1996) The roles of costimulation and Fas in T cell apoptosis and peripheral tolerance. *Immunity*, **4**, 321-8.
Penner, R., Fasolato, C. & Moth, M. (1993) Calcium influx and its control by calcium release. [Review]. *Curr. Op. Neurobiol*., **3**, 368-74.
Petrasch, S., van, T.L., Motulsky, H.J., Brodde, O.E. & Michel, M.C. (1993) Effects of ketotifen on human lymphocytes in vitro and in vivo. *Arzneim.-Forsch..*/*Drug Res.*, **43,** 904-8.
Povoa et al. (1991) *Journal of Internal Medicine,* **229**, 475-7.
Rao, P. & Mufson, R.A. (1994) Human interleukin-3 stimulates a phosphatidylcholine specific phospholipase C and protein kinase C translocation. *Cancer Res.,* **54**, 777-83.
Reed, J.C. (1994) Bcl-2 and the regulation of programmed cell death. *J. Cell Biol*., **124**, 1-6.
Rhee, S.G. (1991) Inositol phospholipids-specific phospholipase C: interaction of the gamma 1 isoform with tyrosine kinase. *TiBS*, **16,** 297-301.
Ribeiro, J.M. & Carson, D.A. (1993) Ca²⁺/Mg²⁺ -dependent endonuclease from human spleen: purification, properties, and role in apoptosis. *Biochem*., **32,** 9129.
Riccardi, V.M. (1987) Mast-cell stabilization to decrease neurofibroma growth. Preliminary experience with ketotifen. *Arch. Dermatol*., **123**, 1011-6.
Riccardi, V. M. (1993) A controlled multiphase trial of ketotifen to minimize neurofibroma-associated pain and itching. *Arch. Dermatol*., **129,** 577-81.
Rodriguez-Tarduchy, G., Prupti, M., Lopez-Rivas, A. & Collins, M.K.L. (1992) Inhibition of apoptosis by calcium ionophores in IL-3-dependent bone marrow cells is dependent upon production of IL-4. J. *Immunol*., **148**, 1416-1422.
Rupp, B. J., L. E. Kahn. Monoclonal antibodies against IgE-associated determinants, hybrid cell lines producing these antibodies, and use therefor. United States Patent No. 4,940,782 issued July 10, 1990.
Schwarze, M.M. & Hawley, R.G. (1995) Prevention of myeloma cell apoptosis by ectopic bcl-2 expression or interleukin 6-mediated up-regulation of bcl-xL. *Cancer Res*., **55,** 2262-5.
Sentman, C.L., Shutter, J.R., Hockenbery, D., Kanagawa, O. & Korsmeyer, S.J. (1991) bcl-2 inhibits multiple forms of apoptosis but not negative selection in thymocytes. *Cell, **67,*** 879-88.
Shibasaki, F. & McKeon, F. (1995) Calcineurin functions in Ca²⁺-activated cell death in mammalian cells. *J. Cell. Biol*., **131**, 735-43.
Siitonen, T., E. R. Savolainen and P. Koistinen. (1994) Expression of the c-kit proto-oncogene in myeloproliferative disorders and myelodysplastic syndromes. *Leuk.* **8,** 631-7.
Slamon, D. J., W. Godolphin, L. A. Jones, J. A. Holt, S. G. Wong, D. E. Keith, W. J. Levin, S. G. Stuart, J. Udove, A. Ullrich (1989) Studies of the HER-2/neu proto-oncogene in human breast and ovarian cancer. *Science.* **244,** 707-12.
Smith, C.A.. Williams, G.T., Kingston, R., Jenkinson, E.J. & Owen, J.J.T. (1989) Antibodies to CD3/T cell receptor complex induce apoptosis in immature T cells in thymic cultures. *Nature*, **337**, 181-4.
Strasser, A., Harris, A.W. & Cory, S. (1991) Bcl-2 transgene inhibi is T cell death and perturbs thymic self-censorship. *Cell*, **67**, 889-99.
Strasser, A., Harris, A.W., Huang, D.C.S, Krammer, P.H. & Cory, S. (1995) Bcl-2 and Fas/APO-1 regulate distinct pathways to lyphocyte apoptosis. *EMBO J*., **14**, 6136-47.
Tadakuma, T., Kizaki, H., Odaka, C., Kubota, R., Ishimura, Y., Yagita, H. & Okumura, K. (1990) CD4+CD8+ thymocytes are susceptible to DNA fragmentation induced by phorbol ester, calcium ionophore and anti-CD3 antibody. *Eur. J. Immunol*., **20**, 779-84.
Takata, M., Homma, Y. & Kurosaki, T. (1995) Requirement of phospholipase C-γ2 activation in surface immunoglobulin M-induced B cell apoptosis. *J. Exp. Med.,* **182**, 907-914.
Taylor, J.D., K. V. Honn. Inhibition of tumor growth and metastasis with calcium channel blocker compounds. United States Patent No. 4,690,935, issued September 1, 1987.
Thor, A. D., D. I. Moore, S. M. Edgerton, E. S. Kawasaki, E. Reihsaus, H. T. Lynch, J. N. Marcus, L. Schwartz, L. C. Chen, B. H. Mayall and a. I. et. (1992) Accumulation of p53 tumor suppressor gene protein: an independent marker of prognosis in breast cancers. *J. Natl. Cancer Inst*. **84**, 845-55.
Tsujimura, T., Furitsu, T., Morimoto, M., Isozaki, K., Nomura, S., Matsuzawa, Y., Kitamura, Y. & Kanakura, Y. (1994) Ligand-independent activation of c-kit receptor tyrosine kinase in a murine mastocytoma cell line P-815 generated by a point mutation. *Blood,* **83,** 2619-26.
Tsujimura, T., T. Furitsu, M. Morimoto, Y. Kanayama, S. Nomura, Y. Matsuzawa, Y. Kitamura and Y. Kanakura. (1995) Substitution of an aspartic acid results in constitutive activation of c-kit receptor tyrosine kinase in a rat tumor mast cell line RBL-2H3. *Int. Arch. Allergy & Immunol.* **106**, 377-85.
Vasilakos, J.P., Ghayur, T., Carroll, R.T., Giegel, D.A., Saunders, J.M., Quintal, L., Keane, K.M. & Shivers, B.D. (1995a) IL-1 beta converting enzyme (ICE) is not required for apoptosis induced by lymphokine deprivation in an IL-2-dependent T cell line. J. *Immunol*., **155**, 3433-42.
Veis, D.J., Sentman, C.L. Bach, E.A. & Korsmeyer, S.J. (1993) Expression of the Bcl-2 protein in murine and human thymocytes and in peripheral T lymphocytes. *J. Immunol*., **151**, 2546-54.
Wang, N. P., H. To, W. H. Lee and E. Y. Lee. (1993) Tumor suppressor activity of RB and p53 genes in human breast carcinoma cells. *Oncogene.* **8,** 279-88.

## Claims

1. A pharmaceutical composition for use as an anti-proliferative agent to inhibit cell growth in a mammal, comprising a Ca²⁺ influx blocker and a factor which normally binds to a receptor of said cell so as to cause Ca²⁺ influx into the cell.

2. A composition of claim 1 wherein binding of said factor to the receptor normally results in activation of phospholipase C.

3. A composition of claim 1 or 2 wherein said Ca²⁺ influx blocker is a non-voltage-gated Ca²⁺ influx blocker.

4. A composition of claim 1, 2 or 3 wherein the Ca²⁺ influx blocker is selected from the group consisting of Ni²⁺, ketotifen, econazole, tenidap, CAl, Cd²⁺, Co²⁺, La³⁺, Mn²⁺, SKF-96365 and cromolyn, or a combination thereof.

5. A composition of claim 1, 2, 3 or 4 wherein said factor is an agonistic antibody to the receptor.

6. A composition of claim 1, 2, 3 or 4 wherein said factor is a natural ligand of the receptor.

7. A composition of claim 1, 2, 3 or 4 wherein said factor is selected from the group SLF, TGF-α, EGF, heregulin, agonists to erbB1, agonists to erbB2, agonists to erbB3, agonists to erbB4, PDGF A, PDGF B, FLT-3 ligand, basic FGF, acidic FGF, enothelin, NGF, VEGF, HGF, agonists to a TCR, agonists to the CD3 receptor, agonists to the FcγRII receptor, agonists to the FcγRIII receptor, agonists to the FcεRI receptor, agonists of G-protein-linked receptors, bombesin, gastrin, gastrin releasing peptide, bradykinin, carbachol, muscarinic receptor agonists, CCK-8, vasopressin, neurokinins, substance P, purinergic receptor agonists, ATP, adenosine, and 1,25-dihydroxyvitamin D₃, and combinations thereof.

8. A composition of claim 7 wherein said factor is SLF.

9. A composition of claim 1 wherein said factor is an antigen of a surface antibody of an immune cell.

10. A composition of claim 1 wherein said factor is an agonist to an IgE.

11. A composition of claim 1 wherein binding of a said factor to the receptor normally, in the absence of a said Ca²⁺ influx blocker, promotes growth, survival and/or activation of said cell.

12. A composition of any preceding claim wherein the Ca²⁺ influx blocker and the factor are provided for separate administration.

13. A composition of any of claims 1 to 11 wherein the Ca²⁺ influx blocker and the factor are provided in a mixture.

14. A pharmaceutical composition for use as an anti-proliferative agent in a mammal, the composition comprising a Ca²⁺ influx blocker and a ligand of a surface receptor of a cell subject to stimulation by the ligand wherein the receptor is selected from the group consisting of a c-kit receptor or a mutant c-kit receptor, a T cell receptor, CD3, FcγRII, FcγRIII, FcεRI, a G-protein-linked receptor, a receptor for bombesin, a receptor for a gastrin releasing peptide, a receptor for bradykinin, a receptor for carbachol, a receptor for a muscarinic molecule, a receptor for CCK-8, a receptor for vasopressin, a receptor for neurokinin, a receptor for substance P, a receptor for a purinergic molecule, a receptor for TGF-α, a receptor for EGF, a receptor for heregulin, a receptor for erbB1, a receptor for erbB2, a receptor for erbB3, a receptor for erbB4, a receptor for PDGF, a receptor for SLF, a receptor for the FLT-3 ligand, a receptor for a basic FGF, a receptor for an acidic FGF, a receptor for enothelin, a receptor for NGF, a receptor for VEGF, and a receptor for HGF.

15. A composition of claim 14 wherein the Ca²⁺ influx blocker and the ligand are provided for separate administration.

16. A composition of claim 14 wherein the Ca²⁺ influx blocker and the ligand are to be administered in a single step.

17. A composition of any preceding claim wherein the Ca²⁺ influx blocker is selected from the group consisting of Ni²⁺, ketotifen, econazole, tenidap, CAI, Cd²⁺, Co²⁺, La³⁺, Mn²⁺, SKF-96365 and cromolyn, or a combination thereof.

18. A composition of any preceding claim, for use as an anti-inflammatory agent.

19. A kit of pharmaceutical compositions for use in inhibiting cell growth in a mammal, the kit comprising a Ca²⁺ influx blocker and a factor which normally binds to a receptor of said cell so as to cause Ca²⁺ influx into the call.

20. A kit of pharmaceutical compositions for use in inhibiting cell growth in a mammal, the kit comprising a Ca²⁺ influx blocker of any of claims 1 to 4 and a factor of any of claims 1 and 5 to 11.

21. A kit of pharmaceutical compositions for use in inhibiting cell growth in a mammal, the kit comprising a Ca²⁺ influx blocker of any of claims 1 to 4 and a ligand of claim 14.

22. Use of a Ca²⁺ influx blocker and a factor capable of stimulating cells with accompanying Ca²⁺ influx for the preparation of a pharmaceutical composition for inducing death of cells in a mammal in need thereof.

23. Use of a Ca²⁺ influx blocker of any of claims 1 to 4 and a factor of any of claims 1 and 5 to 11 for the preparation of a pharmaceutical composition for inducing death of cells in a mammal in need thereof.

24. Use of a Ca²⁺ influx blocker and a ligand of a surface receptor of a cell subject to stimulation by the ligand wherein the receptor is selected from the group consisting of a c-kit receptor or a mutant c-kit receptor, a T cell receptor, CD3, FcγRII, FcγRIII, FcεRI, a G-protein-linked receptor, a receptor for bombesin, a receptor for a gastrin releasing peptide, a receptor for bradykinin, a receptor for carbachol, a receptor for a muscarinic molecule, a receptor for CCK-8, a receptor for vasopressin, a receptor for neurokinin, a receptor for substance P, a receptor for a purinergic molecule, a receptor for TGF-α, a receptor for EGF, a receptor for heregulin, a receptor for erbB1, a receptor for erbB2, a receptor for erbB3, a receptor for erbB4, a receptor for PDGF, a receptor for SLF, a receptor for the FLT-3 ligand, a receptor for a basic FGF, a receptor for an acidic FGF, a receptor for enothelin, a receptor for NGF, a receptor for VEGF, and a receptor for HGF, for the preparation of a pharmaceutical composition for use as an anti-proliferative agent in a mammal.

25. A pharmaceutical composition for use in the treatment of breast cancer, the composition comprising EGF and a Ca²⁺ influx blocker.

26. A pharmaceutical composition for use in the treatment of bone marrow mast cells, the composition comprising SLF and a Ca²⁺ influx blocker.

27. A method for diagnosing susceptibility of diseased mammalian cells to treatment with a Ca²⁺ influx blocker, or a blocker and factor which further activates receptors of the cells, comprising:
assaying whether a sample of tissue contains a level of PLC activity which is elevated in comparison to normal tissue; wherein,
an elevated level of activated PLC indicates the cells are likely to be susceptible to said treatment.

28. The method of claim 27, wherein the step of assaying the sample includes monitoring the amount of a PLC substrate which reacts in the presence of PLC obtained from a predetermined amount of said tissue.

29. The method of claim 28 wherein said PLC substrate is [³H]-PIP₂.

30. A method for screening an agent as a Ca²⁺ influx blocker, the method comprising the steps of:
culturing a first group of cells in the presence of the agent, wherein the cells have an activated receptor which promotes influx of Ca²⁺ into the cells;
culturing a second group of cells in the presence of the agent, wherein the cells of the second group lack an activated receptor which promotes influx of Ca²⁺ into cells of the second group;
ascertaining whether growth of the first group of cells is less than a first predetermined level corresponding to growth of cells of the first group in the absence of the agent; and
ascertaining whether growth of the second group of cells is substantially the same as a second predetermined level corresponding to growth of cells of the second group in the absence of the agent; wherein,
growth of the first group of cells less than the first predetermined level and growth of the second group of cells substantially the same as the second predetermined level indicates that said agent is a Ca²⁺ blocker.

31. The method of claim 30 wherein the receptor of the first group of cells is constitutively activated.

32. The method of claim 30 wherein the receptor of the first group of cells is subject to autocrine stimulation.

33. The method of claim 30 wherein the receptor of the first group of cells is activated by an exogenous agent.

34. The method of claim 33 wherein the agent is a natural ligand of the receptor of the first group of cells.

35. The method of claim 30, 33 or 34 wherein the receptor of the first group of cells and the receptor of the second group of cells is the same receptor.

36. The method of claim 30, 33,34 or 35 wherein the receptor is selected from the group of a c-kit receptor, a receptor for EGF and a receptor for FGF.

37. The method of claim 30, 31, 32, 33, 34, 35 or 36 wherein the cells of the first group and the cells of the second group are human cells.

38. The method of claim 30, 31, 32, 33, 34, 35 or 37 wherein the cells of the first group are mast cells, the cells of the second group are mast cells, the receptor is the c-kit receptor and the first group of cells is cultured in the presence of SLF.

39. The method of claim 30 wherein the first group of cells is transfected with said receptor of the first group of cells.

40. Use of EGF and a Ca²⁺ influx blocker for the preparation of a pharmaceutical composition for treatment of breast cancer.

## Patentansprüche

1. Arzneimittel zur Verwendung als anti-proliferativer Wirkstoff zur Hemmung von Zellwachstum in einem Säuger, umfassend einen Ca²⁺-Einstrom-Blocker und einen Faktor, der normalerweise einen Rezeptor der Zelle bindet, so dass ein Ca²⁺-Einstrom in die Zelle bewirkt wird.

2. Arzneimittel nach Anspruch 1, wobei die Bindung des Faktors an den Rezeptor normalerweise zu einer Aktivierung von Phospholipase C führt.

3. Arzneimittel nach Anspruch 1 oder 2, wobei der Ca²⁺-Einstrom-Blocker ein nicht-spannungsgesteuerter Ca²⁺-Einstrom-Blocker ist.

4. Arzneimittel nach Anspruch 1, 2 oder 3, wobei der Ca²⁺-Einstrom-Blocker ausgewählt ist aus der Gruppe bestehend aus Ni²⁺, Ketotifen, Econazol, Tenidap, CAl, Cd²⁺, Co²⁺, La³⁺, Mn²⁺, SKF-96365 und Cromolyn oder einer Kombination davon.

5. Arzneimittel nach Anspruch 1, 2, 3 oder 4, wobei der Faktor ein agonistischer Antikörper zu dem Rezeptor ist.

6. Arzneimittel nach Anspruch 1, 2, 3 oder 4, wobei der Faktor ein natürlicher Ligand des Rezeptors ist.

7. Arzneimittel nach Anspruch 1, 2, 3 oder 4, wobei der Faktor ausgewählt ist aus der Gruppe SLF, TGF-α, EGF, Heregulin, Agonisten zu erbB1, Agonisten zu erbB2, Agonisten zu erbB3, Agonisten zu erbB4, PDGF A, PDGF B, FLT-3-Ligand, basischem FGF, saurem FGF, Enothelin, NGF, VEGF, HGF, Agonisten zu einem TCR, Agonisten zum CD3-Rezeptor, Agonisten zum FcγRII-Rezeptor, Agonisten zum FcγRIII-Rezeptor, Agonisten zum FcεRI-Rezeptor, Agonisten von G-Protein-gekoppelten Rezeptoren, Bombesin, Gastrin, gastrinfreisetzendem Peptid, Bradykinin, Carbachol, Muscarin-Rezeptor-Agonisten, CCK-8, Vasopressin, Neurokininen, Substanz P, Agonisten zum purinergen Rezeptor, ATP, Adenosin und 1,25-Dihydroxyvitamin D₃ und Kombinationen davon.

8. Arzneimittel nach Anspruch 7, wobei der Faktor SLF ist.

9. Arzneimittel nach Anspruch 1, wobei der Faktor ein Antigen zu einem Oberflächenantikörper einer Immunzelle ist.

10. Arzneimittel nach Anspruch 1, wobei der Faktor ein Agonist zu einem IgE ist.

11. Arzneimittel nach Anspruch 1, wobei die Bindung des Faktors an einen Rezeptor in Abwesenheit des Ca²⁺-Einstrom-Blockers normalerweise Wachstum, Überleben und/oder Aktivierung der Zelle fördert.

12. Arzneimittel nach einem der vorangehenden Ansprüche, wobei der Ca²⁺-Einstrom-Blocker und der Faktor zur getrennten Verabreichung bestimmt sind.

13. Arzneimittel nach einem der Ansprüche 1 bis 11, wobei der Ca²⁺-Einstrom-Blocker und der Faktor in einer Mischung bereitgestellt sind.

14. Arzneimittel zur Verwendung als anti-proliferativer Wirkstoff in einem Säuger, wobei das Arzneimittel einen Ca²⁺-Einstrom-Blocker und einen Liganden eines Oberflächenrezeptors einer Zelle umfasst, wobei die Zelle durch den Liganden stimuliert wird, wobei der Rezeptor ausgewählt ist aus der Gruppe bestehend aus c-Kit-Rezeptor oder einer Mutante des c-Kit-Rezeptors, einem T-Zell-Rezeptor, CD3, FcγRII, FcγRIII, FcεRI, einem G-Protein-gekoppelten Rezeptor, einem Rezeptor für Bombesin, einem Rezeptor für ein gastrinfreisetzendes Peptid, einem Rezeptor für Bradykinin, einem Rezeptor für Carbachol, einem Rezeptor für ein Muscarin-Molekül, einem Rezeptor für CCK-8, einem Rezeptor für Vasopressin, einem Rezeptor für Neurokinin, einem Rezeptor für die Substanz P, einem Rezeptor für ein purinerges Molekül, einem Rezeptor für TGF-α, einem Rezeptor für EGF, einem Rezeptor für Heregulin, einem Rezeptor für erbB1, einem Rezeptor für erbB2, einem Rezeptor für erbB3, einem Rezeptor erbB4, einem Rezeptor für PDGF, einem Rezeptor für SLF, einem Rezeptor für den FLT-3-Liganden, einem Rezeptor für einen basischen FGF, einem Rezeptor für einen sauren FGF, einem Rezeptor für Enothelin, einem Rezeptor für NGF, einem Rezeptor für VEGF und einem Rezeptor für HGF.

15. Arzneimittel nach Anspruch 14, wobei der Ca²⁺-Einstrom-Blocker und der Ligand zur getrennten Verabreichung bestimmt sind.

16. Arzneimittel nach Anspruch 14, wobei der Ca²⁺-Einstrom-Blocker und der Ligand in einem Schritt zu verabreichen sind.

17. Arzneimittel nach einem vorangehenden Anspruch, wobei der Ca²⁺-Einstrom-Blocker ausgewählt ist aus der Gruppe bestehend aus Ni²⁺, Ketotifen, Econazol, Tenidap, CAI, Cd²⁺, Co²⁺, La³⁺, Mn²⁺, SKF-96365 und Cromolyn oder einer Kombination davon.

18. Arzneimittel nach einem vorangehenden Anspruch zur Verwendung als entzündungshemmender Wirkstoff.

19. Kit aus Arzneimitteln zur Verwendung bei der Hemmung von Zellwachstum in einem Säuger, wobei das Kit einen Ca²⁺-Einstrom-Blocker und einen Faktor umfasst, der normalerweise an einen Rezeptor der Zelle bindet, so dass ein Ca²⁺-Einstrom in die Zelle bewirkt wird.

20. Kit aus Arzneimitteln zur Verwendung bei der Hemmung von Zellwachstum in einem Säuger, wobei das Kit einen Ca²⁺-Einstrom-Blocker nach einem der Ansprüche 1 bis 4 und einen Faktor nach einem der Ansprüche 1 und 5 bis 11 umfasst.

21. Kit aus Arzneimitteln zur Verwendung bei der Hemmung von Zellwachstum in einem Säuger, wobei das Kit einen Ca²⁺-Einstrom-Blocker nach einem der Ansprüche 1 bis 4 und einen Liganden nach Anspruch 14 umfasst.

22. Verwendung eines Ca²⁺-Einstrom-Blockers und eines Faktors, der in der Lage ist Zellen mit begleitendem Ca²⁺-Einstrom zu stimulieren, für die Herstellung eines Arzneimittels zur Induzierung von Zelltod in einem Säuger, der dessen bedarf.

23. Verwendung eines Ca²⁺-Einstrom-Blockers nach einem der Ansprüche 1 bis 4 und eines Faktors nach einem der Ansprüche 1 und 5 bis 11 für die Herstellung eines Arzneimittels zur Induzierung von Zelltod in einem Säuger, der dessen bedarf.

24. Verwendung eines Ca²⁺-Einstrom-Blockers und eines Liganden eines Oberflächenrezeptors einer Zelle, die durch den Liganden stimuliert wird, wobei der Rezeptor ausgewählt ist aus der Gruppe bestehend aus c-Kit-Rezeptor oder einer Mutante des c-Kit-Rezeptors, einem T-Zell-Rezeptor, CD3, FcγRII, FcγRIII, FcεRI, einem G-Protein-gekoppelten Rezeptor, einem Rezeptor für Bombesin, einem Rezeptor für ein gastrinfreisetzendes Peptid, einem Rezeptor für Bradykinin, einem Rezeptor für Carbachol, einem Muscarinmolekül-Rezeptor, einem Rezeptor für CCK-8, einem Rezeptor für Vasopressin, einem Rezeptor für Neurokinin, einem Rezeptor für die Substanz P, einem Rezeptor für ein purinerges Molekül, einem Rezeptor für TGF-α, einem Rezeptor für EGF, einem Rezeptor für Heregulin, einem Rezeptor für erbB1, einem Rezeptor für erbB2, einem Rezeptor für erbB3, einem Rezeptor für erbB4, einem Rezeptor für PDGF, einem Rezeptor für SLF, einem Rezeptor für den FLT-3-Ligand, einem Rezeptor für einen basischen FGF, einem Rezeptor für einen sauren FGF, einem Rezeptor für Enothelin, einem Rezeptor für NGF, einem Rezeptor für VEGF und einem Rezeptor für HGF für die Herstellung eines Arzneimittels zur Verwendung als ein anti-proliferativer Wirkstoff in einem Säuger.

25. Arzneimittel zur Verwendung bei der Behandlung von Brustkrebs, wobei das Arzneimittel EGF und einen Ca²⁺-Einstrom-Blocker umfasst.

26. Arzneimittel zur Verwendung bei der Behandlung von Knochenmarksmastzellen, wobei das Arzneimittel SLF und einen Ca²⁺-Einstrom-Blocker umfasst.

27. Verfahren zur Diagnose der Empfänglichkeit von erkrankten Säugerzellen für die Behandlung mit einem Ca²⁺-Einstrom-Blocker oder einem Blocker und einem Faktor, der Rezeptoren der Zelle weiter aktiviert, umfassend:
Untersuchen, ob eine Gewebeprobe eine PLC-Aktivität aufweist, deren Höhe im Vergleich zu normalem Gewebe erhöht ist; wobei
eine Erhöhung von aktiviertem PLC anzeigt, dass die Zellen für die Behandlung wahrscheinlich empfänglich sind.

28. Verfahren nach Anspruch 27, wobei der Schritt des Untersuchens der Probe die Erfassung der Menge eines PLC-Substrats umfasst, das in Anwesenheit von PLC, das aus einer vorherbestimmten Menge des Gewebes erhalten wurde, reagiert.

29. Verfahren nach Anspruch 28, wobei das PLC-Substrat [³H]-PIP₂ ist.

30. Verfahren zum Screenen eines als Ca²⁺-Einstrom-Blocker wirkenden Wirkstoffs, wobei das Verfahren die Schritte umfasst:
Züchten einer ersten Gruppe von Zellen in Anwesenheit des Wirkstoffs, wobei die Zellen einen aktivierten Rezeptor umfassen, der einen Einstrom von Ca²⁺ in die Zellen fördert;
Züchten einer zweiten Gruppe von Zellen in Anwesenheit des Wirkstoffs, wobei den Zellen der zweiten Gruppe ein aktivierter Rezeptor fehlt, der einen Einstrom von Ca²⁺ in Zellen der zweiten Gruppe fördert;
Feststellen, ob das Wachstum der ersten Gruppe von Zellen geringer ist als ein erster, vorherbestimmter Grad an Wachstum von Zellen der ersten Gruppe in Abwesenheit des Wirkstoffs; und
Bestimmen, ob das Wachstum der zweiten Gruppe von Zellen im Wesentlichen gleich ist wie ein zweiter, vorherbestimmter Grad an Wachstum von Zellen der zweiten Gruppe in Abwesenheit des Wirkstoffs; wobei
ein Wachstum der ersten Gruppe von Zellen, das geringer ist als der erste, vorherbestimmte Grad, und ein Wachstum der zweiten Gruppe von Zellen, das im Wesentlichen gleich ist wie der zweite, vorherbestimmte Grad, anzeigt, dass der Wirkstoff ein Ca²⁺-Blocker ist.

31. Verfahren nach Anspruch 30, wobei der Rezeptor der ersten Gruppe von Zellen konstitutiv aktiviert wind.

32. Verfahren nach Anspruch 30, wobei der Rezeptor der ersten Gruppe von Zellen einer autokrinen Stimulation unterliegt.

33. Verfahren nach Anspruch 30, wobei der Rezeptor der ersten Gruppe von Zellen durch einen exogenen Wirkstoff aktiviert wird.

34. Verfahren nach Anspruch 33, wobei der Wirkstoff ein natürlicher Ligand des Rezeptors der ersten Gruppe von Zellen ist.

35. Verfahren nach Anspruch 30, 33 oder 34, wobei der Rezeptor der ersten Gruppe von Zellen und der Rezeptor der zweiten Gruppe von Zellen der gleiche Rezeptor ist.

36. Verfahren nach Anspruch 30, 33, 34 oder 35, wobei der Rezeptor ausgewählt ist aus der Gruppe aus einem c-Kit-Rezeptor, einem Rezeptor für EGF und einem Rezeptor für FGF.

37. Verfahren nach Anspruch 30, 31, 32, 33, 34, 35 oder 36, wobei die Zellen der ersten Gruppe und die Zellen der zweiten Gruppe menschliche Zellen sind.

38. Verfahren nach Anspruch 30, 31, 32, 33, 34, 35 oder 37, wobei die Zellen der ersten Gruppe Mastzellen sind, die Zellen der zweiten Gruppe Mastzellen sind, der Rezeptor der c-Kit-Rezeptor ist und die erste Gruppe von Zellen in Anwesenheit von SLF gezüchtet ist.

39. Verfahren nach Anspruch 30, wobei die erste Gruppe der Zellen mit dem Rezeptor der ersten Gruppe von Zellen transfiziert ist.

40. Verwendung von EGF und einem Ca²⁺-Einstrom-Blocker für die Herstellung eines Arzneimittels zur Behandlung von Brustkrebs.

## Revendications

1. Composition pharmaceutique destinée à être utilisée comme agent anti-prolifératif pour inhiber la croissance cellulaire chez un mammifère, comprenant un bloqueur d'influx de Ca²⁺ et un facteur qui se lie normalement à un récepteur de ladite cellule de façon à provoquer un influx de Ca²⁺ dans la cellule.

2. Composition selon la revendication 1, dans laquelle la liaison dudit facteur au récepteur entraine normalement l'activation de la phospholipase C.

3. Composition selon la revendication 1 ou 2, dans laquelle ledit bloqueur d'influx de Ca²⁺ est un bloqueur de l'influx de Ca²⁺ non voltage-dépendant.

4. Composition selon la revendication 1, 2 ou 3, dans laquelle le bloqueur d'influx de Ca²⁺ est sélectionné parmi le groupe constitué du Ni²⁺, du kétotifène, de l'éconazole, du ténidap, du CAI, du Cd²⁺, du Co²⁺, du La³⁺, du Mn²⁺, du SKF-96365 et de la cromolyne, ou une combinaison de ceux-ci.

5. Composition selon la revendication 1, 2, 3 ou 4, dans laquelle ledit facteur est un anticorps agoniste du récepteur.

6. Composition selon la revendication 1, 2, 3 ou 4, dans laquelle ledit facteur est un ligand naturel du récepteur.

7. Composition selon la revendication 1, 2, 3 ou 4, dans laquelle ledit facteur est sélectionné parmi le groupe SLF, TGF-α, EGF, héréguline, agonistes de erbB1, agonistes de erbB2, agonistes de erbB3, agonistes de erbB4, PDGF A, PDGF B, ligand de FLT-3, FGF basique, FGF acide, énothéline, NGF, VEGF, HGF, agonistes d'un TCR, agonistes du récepteur CD3, agonistes du récepteur FcγRII, agonistes du récepteur FcγRIII, agonistes du récepteur FcεRI, agonsites des récepteurs couplés à une protéine G, bombésine, gastrine, peptide libérant la gastrine, bradykinine, carbachol, agonsites d'un récepteur muscarinique, CCK-8, vasopressine, neurokinines, substance P, agonistes d'un récepteur purinergique, ATP, adénosine, et 1,25-dihydroxyvitamin D₃ et des combinaisons de ceux-ci.

8. Composition selon la revendication 7, dans laquelle ledit facteur est SLF.

9. Composition selon la revendication 1, dans laquelle ledit facteur est un antigène d'un anticorps de surface d'une cellule immune.

10. Composition selon la revendication 1, dans laquelle ledit facteur est un agoniste dune IgE.

11. Composition selon la revendication 1, dans laquelle la liaison d'un dit facteur au récepteur, en absence d'un dit bloqueur d'influx de Ca²⁺, favorise normalement la croissance, la survie et/ou l'activation d'une dite cellule.

12. Composition selon l'une des revendications précédentes, dans laquelle le bloqueur d'influx de Ca²⁺ et le facteur sont fournis pour administration séparée.

13. Composition selon l'une des revendications 1 à 11, dans laquelle le bloqueur d'influx de Ca²⁺ et le facteur sont fournis sous forme de mélange.

14. Composition pharmaceutique destinée à être utilisée comme agent anti-prolifératif chez un mammifère, la composition comprenant un bloqueur d'influx de Ca²⁺ et un ligand d'un récepteur de surface d'une cellule sujette à la stimulation par le ligand, dans laquelle le récepteur est sélectionné parmi le groupe constitué d'un récepteur c-kit ou d'un récepteur c-kit muté, un récepteur de cellule T, CD3, FcγRII, FcγRIII, FcεRI, un récepteur couplé à une protéine G, un récepteur à la bombésine, un récepteur à un peptide libérant la gastrine, un récepteur à la bradykinine, un récepteur au carbachol, un récepteur à une molécule muscarinique, un récepteur au CCK-8, un récepteur à la vasopressine, un récepteur à la neurokinine, un récepteur à la substance P, un récepteur à une molécule purinergique, un récepteur au TGF-α, un récepteur à l'EGF, un récepteur à l'héréguline, un récepteur à erbB1, un récepteur à erbB2, un récepteur à erbB3, un récepteur à erbB4, un récepteur au PDGF, un récepteur au SLF, un récepteur au ligand de FLT-3, un récepteur à un FGF basique, un récepteur à un FGF acide, un récepteur à l'énothéline, un récepteur au NGF, un récepteur au VEGF, et un récepteur au HGF.

15. Composition selon la revendication 14, dans laquelle le bloqueur d'influx de Ca²⁺ et le facteur sont fournis pour administration séparée.

16. Composition selon la revendication 14, dans laquelle le bloqueur d'influx de Ca²⁺ et le facteur sont administrés en une seule étape.

17. Composition selon l'une des revendications précédentes, dans laquelle le bloqueur d'influx de Ca²⁺ est sélectionné parmi le groupe constitué du Ni²⁺, du kétotifène, de l'éconazole, du ténidap, du CAl, du Cd²⁺, du Co²⁺, du La³⁺, du Mn²⁺, du SKF-96365 et de la cromolyne, ou une combinaison de ceux-ci.

18. Composition selon l'une des revendications précédentes, pour une utilisation comme agent anti-inflammatoire.

19. Kit de compositions pharmaceutiques destiné à inhiber la croissance cellulaire chez un mammifère, le kit comprenant un bloqueur d'influx de Ca²⁺ et un facteur qui se lie normalement à un récepteur de ladite cellule de façon à provoquer un influx de Ca²⁺ dans la cellule.

20. Kit de compositions pharmaceutiques destiné à inhiber la croissance cellulaire chez un mammifère, le kit comprenant un bloqueur d'influx de Ca²⁺ selon l'une des revendications 1 à 4 et un facteur selon l'une des revendications 1 et 5 à 11.

21. Kit de compositions pharmaceutiques destiné à inhiber la croissance Cellulaire chez un mammifère, le kit comprenant un bloqueur d'influx de Ca²⁺ selon l'une des revendications 1 à 4 et un ligand selon la revendication 14.

22. Utilisation d'un bloqueur d'influx de Ca²⁺ et d'un facteur capable de stimuler des cellules avec un influx de Ca²⁺ associé pour la préparation d'une composition pharmaceutique pour induire la mort de cellules chez un mammifère le nécessitant.

23. Utilisation d'un bloqueur d'influx de Ca²⁺ selon l'une des revendications 1 à 4 et d'un facteur selon l'une des revendications 1 et 5 à 11 pour la préparation d'une composition pharmaceutique pour induire la mort des cellules chez un mammifère le nécessitant.

24. Utilisation d'un bloqueur d'influx de Ca²⁺ et d'un ligand d'un récepteur de surface d'une cellule sujette à la stimulation par le ligand, dans laquelle le récepteur est sélectionné parmi le groupe constitué d'un récepteur c-kit ou d'un récepteur c-kit muté, un récepteur de cellule T, CD3, FcγRII, FcγRIII, FcεRI, un récepteur couplé à une protéine G, un récepteur à la bombésine, un récepteur à un peptide libérant la gastrine, un récepteur à la bradykinine, un récepteur au carbachol, un récepteur à molécules muscariniques, un récepteur au CCK-8, un récepteur à la vasopressine, un récepteur à la neurokinine un récepteur à la substance P, un récepteur à une molécule purinergique, un récepteur au TGF-α, un récepteur à l'EGF, un récepteur à l'héréguline, un récepteur à erbB1, un récepteur à erbB2, un récepteur à erbB3, un récepteur à erbB4, un récepteur au PDGF, un récepteur au SLF, un récepteur au ligand de FLT-3, un récepteur à un FGF basique, un récepteur à un FGF acide, un récepteur à une énothéline, un récepteur au NGF, un récepteur au VEGF, et un récepteur au HGF, pour la préparation d'une composition pharmaceutique destinée à être utilisée comme agent anti-prolifératif chez un mammifère.

25. Composition pharmaceutique destinée au traitement du cancer du sein, la composition comprenant EGF et un bloqueur d'influx de Ca²⁺.

26. Composition pharmaceutique destinée au traitement des mastocytes de la moelle osseuse, la composition comprenant SLF et un bloqueur d'influx de Ca²⁺.

27. Méthode pour diagnostiquer la sensibilité de cellules mammifères malades au traitement par un bloqueur d'influx de Ca²⁺, ou un bloqueur et un facteur qui active en outre les récepteurs des cellules, comprenant :
tester si un échantillon de tissu contient un niveau d'activité PLC augmenté en comparaison à celui d'un tissu normal ;
un niveau élévé d'activé PLC indiquant que les cellules sont probablement sensibles audit traitement.

28. Méthode selon la revendication 27, dans laquelle l'étape de test de l'échantillon inclut le suivi de la quantité d'un substrat de PLC qui réagit en présence de PLC obtenue à partir dune quantité prédéterminée dudit tissu.

29. Méthode selon la revendication 28, dans laquelle ledit substrat de PLC est [³H]-PIP₂.

30. Méthode de criblage d'un agent comme bloqueur d'influx de Ca²⁺, la méthode comprenant les étapes de
cultiver un premier groupe de cellules en présence de l'agent, les cellules ayant un récepteur activé qui favorise l'influx de Ca²⁺ dans les cellules ;
cultiver un second groupe de cellules en présence de l'agent, les cellules du second groupe étant dénuées de récepteur activé qui favorise l'influx de Ca²⁺ dans des cellules du second groupe ;
déterminer si la croissance du premier groupe de cellules est inférieure à un premier niveau prédéterminé correspondant à la croissance des cellules du premier groupe en absence de l'agent ; et
déterminer si la croissance du second groupe de cellules est sensiblement la même qu'un second niveau prédéterminé correspondant à la croissance des cellules du second groupe en absence de l'agent;
une croissance du premier groupe de cellules inférieur au premier niveau prédéterminé et une croissance du second groupe de cellules sensiblement la même que le second niveau prédéterminé indiquant que l'agent est un bloqueur de Ca²⁺.

31. Méthode selon la revendication 30, dans laquelle le récepteur du premier groupe de cellules est constitutivement activé.

32. Méthode selon la revendication 30, dans laquelle le récepteur du premier groupe de cellules est sujet à une stimulation autocrine.

33. Méthode selon la revendication 30, dans laquelle le récepteur du premier groupe de cellules est activé par un agent exogène.

34. Méthode selon la revendication 33, dans laquelle l'agent est un ligand naturel du récepteur du premier groupe de cellules.

35. Méthode selon la revendication 30, 33 ou 34, dans laquelle le récepteur du premier groupe de cellules et le récepteur du second groupe de cellules est le même récepteur.

36. Méthode selon la revendication 30, 33, 34 ou 35, dans laquelle le récepteur est sélectionné parmi le groupe d'un récepteur c-kit, un récepteur à l'EGF et un récepteur au FGF.

37. Méthode selon la revendication 30, 31, 32 33, 34, 35 ou 36, dans laquelle les cellules du premier groupe et les cellules du second groupe sont des cellules humaines.

38. Méthode selon la revendication 30, 31, 32 33, 34, 35 ou 37, dans laquelle les cellules du premier groupe sont des mastocytes, les cellules du second groupe sont des mastocytes, le récepteur est le récepteur c-kit et le premier groupe de cellules est cultivé en présence de SLF.

39. Méthode selon la revendication 30, dans laquelle le premier groupe de cellules est transfecté avec ledit récepteur du premier groupe de cellules.

40. Utilisation de EGF et d'un bloqueur d'influx de Ca²⁺ pour la préparation d'une composition pharmaceutique pour le traitement du cancer du sein.
